# EUROPÄISCHE PATENTANMELDUNG

(11) **EP 2 647 372 A1**
(43) Veröffentlichungstag der Anmeldung: **09.10.2013**
(21) Anmeldenummer: 13174848.5
(22) Anmeldetag: 14.08.2009
(51) Int. Cl.: A61K 31/045, A61K 31/35, A61P 11/00, A61P 11/06, A61P 11/08

(54) **Monoterpene für die Behandlung von Atemwegserkrankungen, insbesondere bronchopulmonalen Erkrankungen**

(30) Priorität: 04.09.2008 DE 102008045702; 12.09.2008 DE 102008047221
(62) Teilanmeldung aus: 09777903.7
(71) Anmelder: Maria Clementine Martin Klosterfrau Vertriebsgesellschaft mbH, 50670 Köln (DE)
(72) Erfinder: Greve, Harald, Dr., 40545 Düsseldorf (DE)
(74) Vertreter: Von Rohr

(57) **Zusammenfassung**

Die Erfindung betrifft die gemeinsame Verwendung mindestens eines systemisch, insbesondere peroral applizierbaren Monoterpens einerseits sowie mindestens eines topisch, insbesondere inhalativ applizierbaren Atemwegstherapeutikums andererseits zur prophylaktischen und/oder therapeutischen Behandlung, insbesondere Kombinationstherapie und/oder Co-Medikation, von Atemwegserkrankungen, insbesondere bronchopulmonalen Erkrankungen. Durch die gemeinsame Verwendung des systemischen Monoterpens mit dem topischen, insbesondere inhalativen Atemwegstherapeutikum gelingt es, zum einen die Wirkung bzw. Effizienz des topischen bzw. inhalativen Atemwegstherapeutikums signifikant, insbesondere in synergistischer Weise, zu steigern und zum anderen dessen erforderliche Dosis signifikant zu reduzieren, verbunden mit den damit einhergehenden Vorteilen (z. B. Vermeidung bzw. Reduzierung von Nebenwirkungen etc.).

## Beschreibung

Die vorliegende Erfindung betrifft das medizinische Gebiet der Atemwegserkrankungen, insbesondere der bronchopulmonalen Erkrankungen, und deren Behandlung.

Insbesondere betrifft die vorliegende Erfindung die gemeinsame Verwendung von systemisch, insbesondere peroral applizierbaren Monoterpenen einerseits zusammen mit topisch, insbesondere inhalativ applizierbaren Atemwegstherapeutika andererseits zur prophylaktischen und/oder therapeutischen Behandlung, insbesondere Kombinationstherapie oder Co-Medikation, von Atemwegserkrankungen bzw. bronchopulmonalen Erkrankungen.

Des weiteren betrifft die vorliegende Erfindung ein Kombinationstherapeutikum, insbesondere in Form eines Kit, bzw. eine Kombinationstherapie für die prophylaktische und/oder therapeutische Behandlung von bronchopulmonalen Erkrankungen bzw. Atemwegserkrankungen unter Verwendung einer Co-Medikation von systemisch applizierbaren, insbesondere peroralen Monoterpenen einerseits und topisch applizierbaren, insbesondere inhalativen Atemwegstherapeutika andererseits.

Unter dem Begriff der Atemwegserkrankungen ist eine allgemeine Bezeichnung zu verstehen, welche sämtlichen, insbesondere entzündlichen Erkrankungen der oberen wie unteren Atemwege bezeichnet, wobei hierunter akute wie chronische Krankheitszustände zu subsumieren sind. Beispiele für Atemwegserkrankungen der oberen Atemwege sind z. B. Entzündungen der Nebenhöhlen (z. B. Rhinosinusitis), während Beispiele für Atemwegserkrankungen der unteren Atemwege z. B. Asthma bronchiale, Bronchitis und COPD sind.

Der Begriff der "bronchopulmonalen Erkrankungen" ist eine generische Bezeichnung insbesondere für sämtliche entzündlichen wie nichtentzündlichen Erkrankungen der unteren Atemwege (d. h. der bronchialen wie pulmonalen Atemwege), hierunter insbesondere Asthma bronchiale, Bronchitis sowie chronische obstruktive Lungenerkrankungen (sogenannte "COPD" bzw. "Chronic Obstructive Pulmonary Disease"), und wird im Rahmen der vorliegenden Erfindung synonym zu dem Begriff der sogenannten "Atemwegserkrankungen der unter Atemwege" verwendet.

Asthma bronchiale, oft vereinfachend auch nur als Asthma bezeichnet, ist eine chronische entzündliche Erkrankung der Atemwege mit dauerhaft bestehender bronchialer Überempfindlichkeit bzw. Hyperreaktivität, Entzündung der Bronchien sowie mangelnder bronchialer Reinigung (Clearance), wobei infolge der Bronchialobstruktion anfallsweise Atemnot auftreten kann, wobei grundsätzlich zwischen nichtallergischem (intrinsischem) und allergischem (extrinsischem) Asthma differenziert wird; daneben kennt man auch Mischtypen von allergischem und nichtallergischem Asthma als auch Mischtypen von Asthma und COPD. Je nach Schweregrad und den damit einhergehenden Krankheitssymptomen klassifiziert man gemäß den sogenannten Leitlinien nach GINA 2006 ("Global Initiative for Asthma") die verschiedenen Asthma-Krankheitsstadien von GINA I bis IV, wobei die Stufe 1 intermittierendes Asthma, die Stufe 2 leichtgradiges Asthma, die Stufe 3 mittelgradiges Asthma und schließlich die Stufe 4 schweres Asthma bezeichnet. In der neuen Klassifikation (GINA 2007) wird gegen die Asthmakontrolle in den Vordergrund gestellt, und es wird somit unterschieden zwischen kontrolliertem, partiell kontrolliertem und unkontrolliertem Asthma.

Als Bronchitis wird dagegen allgemein die Entzündung der Bronchien, insbesondere der Bronchialschleimhaut, bezeichnet, wobei zwischen akuter Bronchitis einerseits und chronischer Bronchitis andererseits unterschieden wird. Die chronische Bronchitis ist gemäß Weltgesundheitsorganisation (WHO) definiert als Husten und Auswurf an den meisten Tagen während mindestens drei Monaten in zwei aufeinanderfolgenden Jahren und gehört zu den häufigsten chronischen Erkrankungen überhaupt (ca. 15 bis 25 %) mit infolgedessen großer Relevanz aus gesundheitsökonomischer Sicht. Im Fall der chronisch obstruktiven Bronchitis besteht eine dauerhafte Bronchialobstruktion, die sich meistens aus einer chronischen Bronchitis entwickelt.

Der Begriff der chronischen obstruktiven Lungenerkrankung bzw. COPD ("Chronic Obstructive Pulmonary Disease") wird als Sammelbegriff für die chronisch obstruktive Bronchitis und das Lungenemphysem verwendet, wobei der Begriff "obstruktiv" das typische Merkmal der dauerhaften Bronchialverengung charakterisiert. Dabei klassifiziert man je nach Schweregrad und den damit einhergehenden Krankheitssymptomen gemäß den sogenannten Leitlinien nach GOLD ("Global Initiative for Chronic Obstructive Lung Disease") die verschiedenen COPD-Krankheitsstadien von GOLD I bis IV.

Bei der Behandlung von bronchopulmonalen Erkrankungen, insbesondere von Atemwegserkrankungen der vorgenannten Art, kommen bei mildem bis mittlerem Schweregrad oftmals topische bzw. lokale, insbesondere inhalative bzw. inhalierbare Atemwegstherapeutika zum Einsatz. Hierzu zählen beispielsweise inhalative Bronchodilatatoren und Bronchospasmolytika, wie inhalative Beta-2-Sympathomimetika, inhalative Anticholinergika, inhalative Kortikosteroide oder dergleichen. Infolge ihrer nur topischen bzw. lokalen Wirkung können die vorgenannten inhalativen Atemwegstherapeutika oftmals nicht die gewünschte therapeutische Wirkung entfalten, so daß zumeist relativ hohe Dosismengen verabreicht werden müssen, um den gewünschten therapeutischen Erfolg zu erzielen, oder aber in schwerwiegenderen Fällen systemische Therapeutika, insbesondere auf Kortikosteroidbasis, bedarfsweise oder sogar dauerhaft hinzugezogen werden müssen. Infolge der hohen einzusetzenden und erforderlichen Dosismengen werden zudem vielfach unerwünschte Nebenwirkungen beobachtet. Bisweilen wird auch eine nicht ausreichende Sensitivität der vorgenannten inhalativen Atemwegstherapeutika in bezug auf die zu behandelnden Atemwegserkrankungen beobachtet. Schließlich ist mit einer rein topischen, insbesondere inhalativen Therapierung der vorgenannten Atemwegserkrankungen insbesondere der Nachteil verbunden, daß die Anwendung inhalativer Atemwegstherapeutika nicht immer zu einer ausreichenden Deposition in den peripheren Atemwegen führen, da kleinste Atemwege, wie beispielsweise die terminalen und respiratorischen Bronchioli, im allgemeinen durch eine inhalative Therapie nicht erreicht werden können, insbesondere bei Einschränkung in der Lungenfunktion, wie z. B. bei schwerer COPD.

So kommen beispielsweise sowohl beim Asthma bronchiale als auch bei der COPD - neben einer Grundtherapie mit sogenannten Bronchodilatatoren - ab einem gewissen Schweregrad auch topische bzw. lokale, insbesondere inhalative bzw. inhalierbare Kortikosteroide zum Einsatz. Daneben kommen bei schwerem Asthma bronchiale entsprechend GINA IV zusätzlich auch systemische, insbesondere perorale Kortikosteroide zum Einsatz.

Inhalierbare bzw. inhalative Kortikosteroide, insbesondere inhalierbare bzw. inhalative Glukokortikoide, welche synonym auch als "Inhalative Corticosteroids" oder einfach nur mit dem Akronym "ICS" bezeichnet werden, gehören zu den wichtigsten Therapeutika zur topischen bzw. lokalen, insbesondere inhalativen Behandlung entzündlicher Atemwegserkrankungen, insbesondere bei Asthma bronchiale und COPD. Das Wirkprinzip besteht bei regelmäßiger Inhalation in einer primär topischen bzw. lokalen Deposition in den Atemwegen, verbunden mit einer zugleich effektiven Entzündungshemmung durch relativ kleine Steroidmengen.

Auf lokaler bzw. topischer Ebene reduzieren inhalierbare Kortikosteroide, insbesondere Glukokortikoide, die Atemwegsentzündung durch die Hemmung von Zytokinen und Arachidonsäure-Metaboliten (AA-Metaboliten), welche aus aktivierten Atemwegsepithelzellen und distalen, die Atemwege auskleidenden, sogenannten alveolaren Makrophagen freigesetzt werden. In Abhängigkeit von der inhalierten Noxe und der genetischen Disposition gelangen durch die verschiedenen, vorgenannten chemotaktisch und vasodilatierend wirkenden Mediatoren unterschiedliche weiße Blutzellen in die Atemwege und verursachen eine entweder eosinophile Zellinfiltration im Fall des Asthma bronchiale oder eine primär granulozytäre Zellinfiltration im Fall der COPD. Diese Zellinfiltration ist als wichtige Determinante zur Entwicklung der Atemwegsentzündung bei Asthma bronchiale und der Bronchitis bekannt.

Nach den aktuellen internationalen und nationalen Therapierichtlinien werden jedoch ICS nicht in den Frühstadien der Atemwegserkrankungen eingesetzt, sondern insbesondere erst bei mildem persistierendem Asthma (GINA II) und bei mittel- bis schwergradiger COPD (GOLD III und IV), wobei zumeist eine dauerhafte Therapie erforderlich ist [vgl. hierzu z. B.: (1) Global Initiative for Chronic Obstructive Lung Disease, GOLD 2004, National Institute of Health NIH and National Heart, Lung and Blood Institute NHLBI, Bethesda, USA, www.goldcopd.com; (2) National Institutes of Health (Hrsg.), Global Strategy for Asthma Management and Prevention, NHLBI/WHO Workshop Report, Bethesda, USA, U.S. Department of Health and Human Services, 2002, Global Initiative for Asthma, GINA 2004, www.gina.com; (3) British Thoracic Society, The British Guidelines on Asthma Management. Asthma in Adults and Schoolchildren, Thorax 2003, 58:1-94; (4) R. Buhl et al., Leitlinie zur Diagnostik und Therapie von Patienten mit Asthma, Deutsche Gesellschaft für Pneumologie und Beatmungsmedizin, Pneumologie 2006, 60:139-183]. Mit dieser anerkannten Therapiestrategie werden die zunehmend bekannten Nebenwirkungen von ICS sowie eine nicht ausreichende Sensitivität von ICS bei COPD berücksichtigt. Aus diesem Grunde bleibt die Therapie mit ICS nur der mittel-und schwergradigen COPD vorbehalten, die jedoch nicht den Progreß bei COPD, sondern nur die Abnahme von Exazerbationen beeinflussen kann (vgl. hierzu z. B.: B. R. Celli, Chronic Obstructive Pulmonary Disease: From Unjustified Nihilism to Evidence-Based Optimism, Proc. Am. Thoracic Soc. 2006, 3:58-65).

Naturgemäß ist für den klinischen Erfolg einer topischen Therapie mit inhalativen Kortikosteroiden, insbesondere Glukokortikoiden, der Grad der Deposition und insbesondere auch die Steroidverteilung in den peripheren Atemwegen von direkter Bedeutung. Aber selbst bei optimalem Einsatz verschiedenster Atemhilfen für Dosieraerosole und Pulverpräparate ist der Therapieerfolg nicht nur durch die Fähigkeit des Patienten, optimal zu inhalieren, sondern vielmehr primär durch das Inhalationsprinzip aufgrund der nicht ausreichenden Behandlung peripherer Atemwege limitiert. Wesentliche Ursachen hierfür sind somit die nicht ausreichende Steroiddeposition in den kleinen Atemwegen (<10⁻⁸ mol/l) und die entsprechend höhere Steroiddeposition auf den Schleimhäuten des Mundes und der Luftröhre. Nach Resorption gelangen hierdurch regelmäßig kleinere Steroidmengen unerwünschtermaßen auch in die Blutbahn und verursachen typische Steroidnebenwirkungen, wie z. B. Hemmung der Cortisolproduktion, Entwicklung einer Osteoporose, Kataraktbildung etc. Diese zunehmend bekannt werdenden Nebenwirkungen limitieren daher bislang auch den therapeutischen Einsatz von ICS bei leichteren Formen der COPD, obgleich ICS, je nach Schweregrad der Atemwegserkrankung, gemäß den Therapierichtlinien noch höher dosiert und in Kombination mit weiteren Therapeutika bis hin zu oralen Glukokortikoiden über einen Zeitraum von zwei bis drei Wochen empfohlen werden. Die eigentliche Ursache hierfür ist auf die nicht ausreichende Deposition von ICS in den peripheren Atemwegen und eine zumindest bei der COPD zusätzlich erforderliche, nichtsteroidale antiinflammatorische Therapie zurückzuführen. Kleinste Atemwege, wie die terminalen und respiratorischen Bronchioli, können derzeit nur durch eine systemische Therapie erreicht werden.

Neben den vorgenannten Therapieansätzen werden, mehr oder weniger traditionell, zur symptomatischen Behandlung beispielsweise bronchitischer Beschwerden und zur Erleichterung des Abhustens bei Hypersekretion, insbesondere bei Erkältungskrankheiten, auch inhalativ anzuwendende ätherische Öle bzw. Ölgemische für einen kürzeren Zeitraum eingesetzt. Hierbei handelt es sich jedoch um eine nichtkausale Therapie, welche insbesondere nur bei leichtgradigen, vor allem akuten Atemwegserkrankungen zum Einsatz kommt, jedoch bei chronischen und insbesondere schwergradigen Atemwegserkrankungen allenfalls unterstützend angewendet werden kann.

In den zu derselben Patentfamilie gehörenden Druckschriften DE 43 19 554 C2, DE 43 19 556 C2 und WO 94/28895 A2 wird eine Kombinationstherapie mit peroral verabreichten Terpenverbindungen, insbesondere 1,8-Cineol oder Menthol, einerseits und ebenfalls systemisch, insbesondere peroral verabreichten Kortikosteroiden zur entzündungshemmenden Behandlung von systemisch steroidpflichtigem chronischem Asthma bronchiale ("GINA IV") beschrieben, wobei der Einsatz der peroral verabreichten Terpenverbindungen im Rahmen einer Dauertherapie den Bedarf systemischer Kortikosteroide reduzieren soll. Die Applikation systemischer Kortikosteroide ist jedoch mit schwerwiegenden Nebenwirkungen verbunden. Aufgrund dieser schwerwiegenden Nebenwirkungen systemischer Kortikosteroide ist die dort beschriebene Kombinationstherapie nur auf schwergradige Formen von Asthma bronchiale ("GINA IV") beschränkt. Inhalative Behandlungskonzepte werden dort weder beschrieben, noch sind diese aufgrund des Schweregrades der dort behandelten Erkrankungen überhaupt in Betracht zu ziehen.

Im Ergebnis liefern die aus dem Stand der Technik bekannten Behandlungsmethoden von bronchopulmonalen Erkrankungen bzw. Atemwegserkrankungen oftmals nicht bzw. allenfalls unter Inkaufnahme von unerwünschten Nebenwirkungen den gewünschten therapeutischen Erfolg. Insbesondere können nach dem Stand der Technik die eingesetzten topischen bzw. lokalen, insbesondere inhalativen bzw. inhalierbaren Atemwegstherapeutika (wie z. B. ICS) nicht immer den gewünschten therapeutischen Effekt bewirken.

Der vorliegenden Erfindung liegt daher die Aufgabe zugrunde, die zuvor geschilderten Nachteile des Standes der Technik zumindest teilweise zu vermeiden oder aber wenigstens abzuschwächen.

Insbesondere soll im Rahmen der vorliegenden Erfindung eine verbesserte und/oder effizientere Therapie zur Behandlung von Atemwegserkrankungen, insbesondere bronchopulmonalen Erkrankungen, bereitgestellt werden.

Weiterhin soll im Rahmen der vorliegenden Erfindung eine verbesserte Wirkeffizienz bzw. ein breiteres Anwendungsspektrum lokal bzw. topisch, insbesondere inhalativ applizierbarer Atemwegstherapeutika der vorgenannten Art (wie z. B inhalative Bronchodilatatoren und/oder Bronchospasmolytika, einschließlich Sympathomimetika, Phosphodiesterasehemmem, Parasympatholytika und/oder Vagolytika, Anticholinergika, Kortikosteroiden etc.) ermöglicht bzw. erreicht werden.

Die Anmelderin hat nun überraschenderweise herausgefunden, daß das zuvor geschilderte Problem dadurch gelöst werden kann, daß man im Rahmen einer Co-Medikation einerseits systemisch, insbesondere peroral mindestens ein Monoterpen (= *systemisch applizierbare Komponente*) und andererseits - gemeinsam bzw. in Kombination hiermit - mindestens ein topisch applizierbares, insbesondere inhalatives Atemwegstherapeutikum (= *topisch applizierbare Komponente*) zu Zwecken der prophylaktischen und/oder therapeutischen Behandlung von Atemwegserkrankungen, insbesondere bronchopulmonalen Erkrankungen, einsetzt.

Vollkommen unerwartet ist in diesem Zusammenhang insbesondere die Tatsache, daß im Rahmen der vorliegenden Erfindung ein systemisch, insbesondere peroral verabreichtes Monoterpen überhaupt imstande ist, mit einem im Unterschied hierzu nur lokal bzw. topisch, insbesondere inhalativ applizierten Atemwegstherapeutikum in Wechselwirkung zu treten und dann auch noch die Wirkung des letzteren unerwarteterweise zu verstärken, vorzugsweise in synergistischer Weise. Auf diese Weise gelingt es, die Wirkeffizienz des topischen, insbesondere inhalativen Atemwegstherapeutikums zu steigern, und es wird infolgedessen ermöglicht, dessen verabreichte Dosismenge signifikant zu reduzieren, einhergehend mit den damit wiederum verbundenen Vorteilen (z. B. Vermeidung bzw. Reduktion von Nebenwirkungen etc.).

Gegenstand der vorliegenden Erfindung - gemäß einem ersten Aspekt der vorliegenden Erfindung - ist somit die Verwendung mindestens eines systemisch applizierbaren, insbesondere peroralen Monoterpens einerseits und mindestens eines topisch applizierbaren, insbesondere inhalativen Atemwegstherapeutikums andererseits zur prophylaktischen und/oder therapeutischen Behandlung, insbesondere Kombinationstherapie und/oder Co-Medikation, von Atemwegserkrankungen, insbesondere bronchopulmonalen Erkrankungen.

Mit anderen Worten ist Gegenstand der vorliegenden Erfindung die Verwendung mindestens eines Monoterpens einerseits und mindestens eines Atemwegstherapeutikums andererseits zur prophylaktischen und/oder therapeutischen Behandlung, insbesondere Kombinationstherapie bzw. Co-Medikation, von Atemwegserkrankungen, insbesondere bronchopulmonalen Erkrankungen, wobei das Monoterpen systemisch, insbesondere peroral appliziert wird und das Atemwegstherapeutikum topisch, insbesondere inhalativ appliziert wird.

Wie die Studien der Anmelderin überraschend zeigen, kann durch die Anwendung eines systemischen, insbesondere peroralen Monoterpens unter Kombinationstherapie mit einem topischen, insbesondere inhalativen Atemwegstherapeutikum im Rahmen der prophylaktischen bzw. therapeutischen Behandlung von Atemwegserkrankungen, insbesondere bronchopulmonalen Erkrankungen, die Effizienz bzw. Wirkung des topischen bzw. inhalativen Atemwegstherapeutikums signifikant, insbesondere in synergistischer Weise, gesteigert werden. Es ist vollkommen überraschend und war nicht zu erwarten, daß das systemisch, insbesondere peroral applizierte Monoterpen die Wirkung bzw. Effizienz des topischen bzw. inhalativen Atemwegstherapeutikums in der aufgefundenen Art und Weise zu steigern imstande ist, da zwei vollkommen unterschiedliche, voneinander getrennte Applikationsarten - nämlich systemisch einerseits und lokal bzw. topisch andererseits - für die beiden zu kombinierenden Wirkstoffe (d. h. Monoterpen einerseits und Atemwegstherapeutikum andererseits) zum Einsatz kommen.

Dieser von der Anmelderin vollkommen überraschend aufgefundene Effekt läßt sich - ohne sich auf eine bestimmte Theorie festlegen zu wollen - möglicherweise darauf zurückführen, daß die systemische bzw. perorale Verabreichung des Monoterpens dazu führt, daß insbesondere infolge von Diffusions- und/oder Exhalationsprozessen ein gewisser Teil des systemisch bzw. peroral applizierten Monoterpens unverändert in das Atemwegsepithel, insbesondere in das Alveolar- und/oder Bronchialepithel, gelangt und dort sozusagen "ausgeatmet" wird und sich infolgedessen in relativ großen Konzentrationen auf und in den Epithelzellen wiederfindet, wo es dann auf das topisch bzw. inhalativ applizierte Atemwegstherapeutikum trifft und hiermit zusammenwirken kann, insbesondere in synergistischer Art und Weise.

Infolge der Lipophilie der Monoterpene wird zudem eine Langzeitspeicherung in den betreffenden Epithelzellen beobachtet, so daß eine langfristige und gleichmäßige Versorgung mit Monoterpenen in den betreffenden Epithelzellen gewährleistet ist, so daß eine langfristige Wechselwirkung mit dem inhalativen Atemwegstherapeutikum gewährleistet ist.

Auf die vorgenannte Art und Weise gelingt es, die Wirkung bzw. Effizienz von topischen bzw. inhalativen Atemwegstherapeutika signifikant, insbesondere in synergistischer Weise, zu steigern und deren eingesetzte Dosismengen signifikant zu reduzieren, verbunden mit den damit einhergehenden Vorteilen (z. B. Vermeidung bzw. Reduzierung von Nebenwirkungen etc.). Gleichzeitig wird durch das Zusammenwirken von Monoterpen einerseits und topischem bzw. inhalativem Atemwegstherapeutikum andererseits die Sensitivität gegenüber dem topischen bzw. inhalativen Atemwegstherapeutikum signifikant gesteigert; folglich kann gegebenenfalls nicht nur die Dosismenge des topischen bzw. inhalativen Atemwegstherapeutikums reduziert werden, sondern unter Umständen auch das betreffende topische bzw. inhalative Atemwegstherapeutikum für bestimmte Atemwegserkrankungen bzw. bestimmte Stadien der vorgenannten Atemwegserkrankungen überhaupt erst zugänglich gemacht werden, bei denen die betreffenden topischen bzw. inhalativen Atemwegstherapeutika infolge der mangelnden Sensitivität bislang gar nicht angewendet werden konnten.

Die Wirkungssteigerung des topischen bzw. inhalativen Atemwegstherapeutikums als solche wiederum läßt sich - wiederum ohne sich auf eine spezielle Theorie festlegen zu wollen - vermutlich auf das von der Anmelderin überraschend aufgefundene steroidartige Wirkungspotential der systemisch eingesetzten Monoterpene erklären, insbesondere im Hinblick auf die Hemmung von Entzündungsmediatoren, welche durch verschiedene infektiöse, allergische und/oder entzündliche Reize gebildet werden und infolge einer Mukus-Hypersekretion eine Zunahme bzw. Exazerbation der jeweiligen Atemwegsentzündungen bewirken. Isolierte Monoterpene (wie z. B. 1,8-Cineol, Menthol etc.) besitzen somit als Vorläufer von Phytosteroiden ein steroidartiges Wirkungspotential; sie bewirken nämlich die Hemmung von Entzündungsmediatoren. Vollständige ätherische Mischöle dagegen stimulieren die Prostaglandinproduktion und zeigen eine verminderte Hemmung der Leukotrien- und Zytokinproduktion im Vergleich zu dem dominierenden Monoterpen als Hauptbestandteil der betreffenden Mischöle; denn die Mischöle enthalten auch solche Substanzen, welche die Zellaktivität und die Mediatorproduktion stimulieren und daher nicht entzündungshemmend wirken, sondern Unverträglichkeitsreaktionen verursachen können, so daß ätherische Mischöle bzw. Ölgemische folglich im allgemeinen die Zellaktivität erhöhen und die Mediatorproduktion und die Schleimbildung induzieren. Im Unterschied hierzu jedoch hemmen isolierte Monoterpene die Mukus-Hypersekretion durch eine Hemmung der Mediatorproduktion; hierin ist kein sekretolytischer, sondern vielmehr ein entzündungshemmender, insbesondere mukolytischer Effekt in den Atemwegen zu sehen. Diese Effekte werden - im Unterschied zu inhalativen Atemwegstherapeutika (z. B. ICS) allein bzw. als Monotherapeutikum - erst mit Monoterpenen in den gesamten Atemwegen, insbesondere in der gesamten Lunge, d. h. auch bis in die Peripherie und die Alveolen, erreicht. Denn Monoterpene werden nach systemischer Gabe, insbesondere in Form von magensaftresistenten, aber dünndarmlöslichen Kapseln, ins Blut aufgenommen und entsprechend ihren physikalischen Eigenschaften in den Alveolen freigesetzt und gelangen somit in die Ausatmungsluft. Hierdurch können Monoterpene gerade auch in den kleinsten, peripher gelegenen Atemwegen - im Unterschied zu inhalativen Atemwegstherapeutika allein - eine entzündungshemmende Wirkung vermitteln.

Auf der Grundlage der vorstehend geschilderten Erkenntnisse ist es der Anmelderin überraschend gelungen, erstmals eine effiziente Kombinationstherapie zur prophylaktischen und/oder therapeutischen Behandlung von Atemwegserkrankungen, insbesondere bronchopulmonalen Erkrankungen, bereitgestellt zu haben, welche im Rahmen einer Co-Medikation die gemeinsame Verwendung mindestens eines systemisch, insbesondere peroral zu applizierenden Monoterpens einerseits und - in Kombination hiermit - mindestens eines topisch, insbesondere inhalativ zu applizierenden Atemwegstherapeutikums andererseits vorschlägt bzw. realisiert.

Es versteht sich von selbst, daß bei der vorangehenden wie noch folgenden Beschreibung der vorliegenden Erfindung solche Ausführungen, welche nur zu einem Erfindungsaspekt gemacht worden sind bzw. werden, selbstverständlich auch für die übrigen Erfindungsaspekte entsprechend gelten, ohne daß dies einer ausdrücklichen Erwähnung bedarf.

Weiterhin versteht es sich von selbst, daß nachfolgend sämtliche Mengen-, Dosis- und Bereichsangaben derart zu verstehen sind, daß erforderlichenfalls, insbesondere einzelfallbedingt oder anwendungsbezogen, hiervon abgewichen werden kann, ohne daß der Rahmen der vorliegenden Erfindung verlassen ist. Dies liegt im Ermessen des Fachmanns.

Es versteht sich für den Fachmann von selbst, daß im Rahmen der erfindungsgemäßen Kombinationstherapie bzw. im Rahmen der erfindungsgemäßen Verwendung das systemisch, insbesondere peroral zu applizierende Monoterpen einerseits und das topisch, insbesondere inhalativ zu applizierende Atemwegstherapeutikum andererseits zeitgleich oder aber zeitversetzt zueinander appliziert werden können. Sowohl im Hinblick auf die systemische als auch im Hinblick auf die topische Komponente kann jeweils eine tägliche Einzelgabe in Betracht kommen, oder aber es können bevorzugterweise die jeweiligen Gesamttagesdosen in zwei oder mehreren Einzelgaben über den Tag verteilt werden; dies zu entscheiden, liegt im Ermessen des Fachmanns.

Was das eingesetzte Monoterpen anbelangt, so kann dieses insbesondere ausgewählt sein aus mono- und bicyclischen Monoterpenen, vorzugsweise aus der Gruppe von monocyclischen Monoterpen-Alkoholen, vorzugsweise Menthol (z. B. L-Menthol), und bicyclischen Epoxy-Monoterpenen, vorzugsweise Limonenoxiden, bevorzugt 1,8-Cineol, sowie deren Mischungen, besonders bevorzugt aus der Gruppe von Menthol und 1,8-Cineol. Erfindungsgemäß ganz besonders bevorzugt wird 1,8-Cineol als Monoterpen verwendet.

Als erfindungsgemäß besonders wirksam hat es sich erwiesen, das Monoterpen als isolierte bzw. einzelne Wirksubstanz einzusetzen (d. h. also keine Mischung verschiedener Monoterpene oder keine ätherischen Öle bzw. Ölgemische zu verwenden), selbstverständlich zusammen mit einem geeigneten pharmazeutischen Träger bzw. Exzipienten und gegebenenfalls zusammen mit weiteren, üblichen phannazeutischen Hilfsstoffen und/oder Additiven. Dennoch ist erfindungsgemäß grundsätzlich nicht ausgeschlossen, zwei oder mehrere Monoterpene gemeinsam, insbesondere in Mischung, einzusetzen, obwohl dies erfindungsgemäß weitaus weniger bevorzugt ist.

Die Terpene sind eine stark heteorogene und sehr große Gruppe von chemischen Verbindungen, die sich biosynthetisch von Isopren- bzw. Isopentenyleinheiten ableiten lassen, wobei die Biosynthese über aktivierte Formen dieser Moleküle, nämlich dem Dimethylallylpyrophosphat (DMAPP) und dem Isopentenylpyrophosphat (IPP) erfolgt; die Einheiten bestehen aus fünf Kohlenstoffatomen (C₅-Einheiten). Es sind über 8.000 Terpene und über 30.000 der nahen verwandten Terpenoide bekannt. Die Terpene zählt man in der Systematik der organischen Chemie zu den Lipiden (sekundäre Naturstoffe). Der gemeinsame Baustein aller Terpene ist das Isopren. Die Terpene zählen zu den sekundären Pflanzenstoffen.

Es sind über 900 Monoterpene bekannt. Alle werden durch Monoterpensynthasen aus Geranylpyrophosphat (2,6-Dimethyloctan) synthetisiert. Im Rahmen der vorliegenden Erfindung kommen bevorzugt mono- und bicyclische Monoterpene zum Einsatz. Die meisten monocyclischen Monoterpene, die sich vom p-Menthan ableiten lassen, weisen ein Cyclohexangerüst auf, während die Bicyclen Caran, Thujan, Pinan, Bornan und Fenchan, aber weiter gefaßt auch Isobornylan und Isocamphan die wichtigsten Stammverbindungen der bicyclischen Monoterpene sind.

Das Menthol wiederum - synonym auch als 2-Isopropyl-5methylcyclohexanol oder aber 5-Methyl-2-(1-methylethyl)-cyclohexan-1-ol bezeichnet - ist ein monocyclischer Monoterpen-Alkohol, wohingegen das 1,8-Cineol - synonym auch als Eucalyptol, Limonen-1,8-oxid, 1,8-Epoxy-pmenthan oder aber 1,3,3-Trimethyl-2-oxabicyclo-[2.2.2]-octan bezeichnet - zu den bicyclischen Epoxy-Monoterpenen, genauer gesagt zu den Limonenoxiden, gehört.

Im Rahmen der vorliegenden Erfindung wird das Monoterpen üblicherweise in einer peroralen Darreichungsform, vorzugsweise in Form von Kapseln, eingesetzt. Erfindungsgemäß besonders bevorzugt ist die Applikation des Monoterpens in Form einer peroralen, magensaftresistenten, aber dünndarmlöslichen Darreichung, insbesondere Kapsel. Ganz besonders bevorzugt sind perorale, magensaftresistente, aber dünndarmlösliche Darreichungsformen, insbesondere Kapseln, welche ein einziges Monoterpen, vorzugsweise 1,8-Cineol, enthalten. Derartige Produkte sind im Handel erhältlich (z. B. Soledum^{®}-Kapseln, vertrieben von der Cassella-med GmbH & Co. KG bzw. der Maria Clementine Martin Klosterfrau Vertriebsgesellschaft mbH, Köln, Bundesrepublik Deutschland).

Im allgemeinen wird das Monoterpen in wirksamen, insbesondere pharmazeutisch wirksamen Mengen eingesetzt. Dabei kann die Dosismenge an Monoterpen in weiten Bereichen variieren. Üblicherweise wird das systemisch, insbesondere peroral applizierte Monoterpen in Tagesdosen von 100 bis 2.000 mg/die, insbesondere 200 bis 1.200 mg/die, vorzugsweise 300 bis 1.000 mg/die, verabreicht. Mit anderen Worten ist das systemisch bzw. peroral zu applizierende Monoterpen im allgemeinen zur Verabreichung in einer Tagesdosis von 100 bis 2.000 mg/die, insbesondere 200 bis 1.200 mg/die, vorzugsweise 300 bis 1.000 mg/die, hergerichtet. Es versteht sich von selbst, daß der Fachmann erforderlichenfalls von den vorgenannten Mengen einzelfallbedingt oder anwendungsbezogen abweichen kann, ohne daß der Rahmen der vorliegenden Erfindung verlassen ist.

Gemäß einer erfindungsgemäß besonders bevorzugten Ausführungsform wird als systemisch bzw. peroral zu applizierendes Monoterpen 1,8-Cineol in Form von magensaftresistenten, aber dünndarmlöslichen Kapseln eingesetzt, vorzugsweise in Tagesdosen im Bereich von 100 bis 2.000 mg/die, insbesondere 200 bis 1.200 mg/die, besonders bevorzugt 300 bis 1.000 mg/die.

Die vorgenannten Tagesdosen können sich vorteilhafterweise auf eine, zwei oder mehrere Einzelgaben verteilen.

Wie zuvor ausgeführt, wird in Kombination mit dem systemisch, insbesondere peroral zu applizierenden Monoterpen ein topisch zu applizierendes Atemwegstherapeutikum als Co-Medikation verabreicht. Wie das erfindungsgemäß eingesetzte Monoterpen, so wird auch das topisch zu applizierende Atemwegstherapeutikum im Rahmen der erfindungsgemäßen Kombinationstherapie in wirksamen, insbesonders pharmazeutisch wirksamen Mengen eingesetzt, wobei der Fachmann in Abhängigkeit von der zu therapierenden Atemwegserkrankung und deren Schweregrad und in Abhängigkeit vom eingesetzten topischen Atemwegstherapeutikum die betreffenden Dosismengen auswählen wird.

Im allgemeinen handelt es sich bei dem erfindungsgemäß eingesetzten, topisch zu applizierenden Atemwegstherapeutikum um ein inhalatives bzw. inhalierbares Atemwegstherapeutikum.

Der Begriff des topisch, insbesondere inhalativ zu applizierenden Atemwegstherapeutikums ist erfindungsgemäß sehr breit zu verstehen und umfaßt insbesondere sämtliche dem Fachmann zur Behandlung von bronchopulmonalen Erkrankungen bzw. Atemwegserkrankungen bekannten Arzneimittel und Arzneimittelkombinationen, welche sich für die topische, insbesondere inhalative Anwendung eignen.

In erfindungsgemäß bevorzugter Weise ist das topisch applizierbare, insbesondere inhalative Atemwegstherapeutikum aus Bronchodilatatoren und Bronchospasmolytika ausgewählt. Der Begriff der Bronchodilatatoren bezeichnet solche Substanzen, welche die Bronchien und Bronchiolen erweitern bzw. dilatieren und auf diese Weise den Atemwegswiderstand herabsetzen, wohingegen der Begriff der Bronchospasmolytika solche Substanzen bezeichnet, welche den Bronialmuskeltonus herabsetzen und zum Teil die Freisetzung von Mediatorsubstanzen aus den Mastzellen hemmen und die mukoziliäre Clearance steigern.

In erfindungsgemäß bevorzugter Weise ist das topisch, insbesondere inhalativ zu applizierende Atemwegstherapeutikum ausgewählt aus der Gruppe von (i) Kortikosteroiden, insbesondere Glukokortikoiden; (ii) Sympathomimetika, insbesondere Betasympathomimetika, vorzugsweise Beta-2-Sympathomimetika; (iii) Phosphodiesterasehemmern; (iv) Parasympatholytika und/oder Vagolytika; (v) Anticholinergika; sowie Mischungen und Kombinationen der vorgenannten Verbindungen.

In erfindungsgemäß ganz bevorzugter Weise ist das topische, insbesondere inhalative Atemwegstherapeutikum ausgewählt aus der Gruppe von Kortikosteroiden, insbesondere Glukokortikoiden, Beta-2-Sympathomimetika und Anticholinergika sowie deren Mischungen und Kombinationen.

Es versteht sich von selbst, daß im Rahmen der erfindungsgemäßen Kombinationstherapie bzw. Verwendung auch Kombinationen von mindestens zwei oder mehreren topischen, insbesondere inhalativen Atemwegstherapeutika der vorgenannten Art in Betracht kommen.

Wie zuvor geschildert, kann gemäß einer erfindungsgemäßen Ausführungsform das topisch applizierbare, insbesondere inhalative Atemwegstherapeutikum ein topisches bzw. inhalatives Kortikosteroid, insbesondere Glukokortikoid, sein. Bei dem topischen bzw. inhalativen Kortikosteroid, insbesondere Glukokortikoid, kann es sich insbesondere um eine Verbindung aus der Gruppe von Beclometason, Mometason, Budesonid, Flunisolid, Fluticason, Triamcinolon und deren physiologisch verträglichen Derivaten, insbesondere Salzen und Estern, sowie Mischungen und Kombinationen handeln.

Die Kortikosteroide sind eine Gruppe von ca. 50 in der Nebennierenrinde gebildeten Steroidhormonen sowie chemisch vergleichbare synthetische Stoffe, wobei alle Kortikosteroide aus dem Ausgangsstoff Cholesterin entstehen und als gemeinsames Grundgerüst das Progestoron (Delta-4-pregnen-3,20-dion) aufweisen. Die Kortikosteroide lassen sich nach ihrer biologischen Wirkung bzw. ihrem Bildungsort in drei Gruppen einteilen, nämlich die Mineralokortikoide, die Glukokortikoide und die Androgene. Die erfindungsgemäß bevorzugt eingesetzten Glukokortikoide zählen somit zu den Kortikosteroiden.

Die Dosismengen, mit welchen die topischen bzw. inhalativen Kortikosteroide, insbesondere Glukokortikoide, eingesetzt werden, können in weiten Bereichen variieren. Das topische bzw. inhalative Kortikosteroid, insbesondere Glukokortikoid, wird üblicherweise in Tagesdosen von 50 bis 1.000 µg/die, insbesondere 75 bis 800 µg/die, besonders bevorzugt 100 bis 600 µg/die, verabreicht bzw. ist insbesondere zur Verabreichung in einer Tagesdosis von 50 bis 1.000 µg/die, insbesondere 75 bis 800 µg/die, besonders bevorzugt 100 bis 600 µg/die, hergerichtet. Es versteht sich von selbst, daß der Fachmann erforderlichenfalls von den vorgenannten Werten im Einzelfall bzw. anwendungsbezogen abweichen kann, ohne daß der Rahmen der vorliegenden Erfindung verlassen ist.

Gemäß einer alternativen Ausführungsform kann das topische bzw. inhalative Atemwegstherapeutikum ein Sympathomimetikum, insbesondere Betasympathomimetikum, vorzugsweise Beta-2-Sympathomimetikum, sein. Erfindungsgemäß besonders bevorzugt ist der Einsatz inhalativer Beta-2-Sympathomimetika.

Der Begriff der Sympathomimetika kennzeichnet solche Substanzen, welche die Wirkung des Sympathikus nachahmen. Speziell die Betasympathomimetika (synonym auch als "Betamimetika" bezeichnet) besitzen überwiegend Wirkung auf die Betarezeptoren. Ganz speziell die erfindungsgemäß bevorzugten Beta-2-Sympathomimetika führen an der glatten Muskulatur (Beta-2-Rezeptoren) zur Erschlaffung und besitzen brochospasmolytische Wirkung.

Bei den erfindungsgemäß bevorzugt eingesetzten inhalativen Beta-2-Sympathomimetika kann es sich entweder um kurzwirkende Betamimetika (SABA = short acting beta agonists) oder um langwirkende Betamimetika (LABA = long acting beta agonists) handeln. Beispiele für kurzwirkende Betamimetika (SABA) sind insbesondere Albuterol, Fenoterol, Hexoprenalin, Levalbuterol, Metaproterenol, Orciprenalin, Pirbuterol, Reproterol, Salbutamol und/oder Terbutalin. Beispiele für langwirkende Betamimetika (LABA) sind insbesondere Salmeterol und/oder Formoterol.

Gemäß einer alternativen Ausführungsform kann es sich bei dem topischen bzw. inhalativen Atemwegstherapeutikum um ein Anticholinergikum handeln. Anticholinergika sind solche Substanzen, welche die Wirkung von Acetylcholin unterdrücken und besitzen gleichermaßen bronchospasmolytische Wirkung. Erfindungsgemäß bevorzugt eingesetzte topische bzw. inhalative Anticholinergika sind Ipratropium, Tiotropium und/oder deren physiologisch verträgliche Derivate, vorzugsweise Salze, besonders bevorzugt Ipratropiumbromid und/oder Tiotropiumbromid.

Es versteht sich für den Fachmann von selbst, daß im Rahmen der erfindungsgemäßen Verwendung bzw. Kombinationstherapie die vorgenannten topischen bzw. inhalativen Atemwegstherapeutika auch miteinander kombiniert werden können.

Weiter kann es im Rahmen der erfindungsgemäßen Verwendung bzw. Kombinationstherapie auch vorgesehen sein, zusätzlich mindestens einen weiteren systemischen, insbesondere peroralen Wirkstoff zu applizieren. Dieser zusätzliche systemische, insbesondere perorale Wirkstoff kann insbesondere ausgewählt sein aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; sowie deren Mischungen und Kombinationen.

Im Rahmen der erfindungsgemäßen Verwendung können beliebige bronchopulmonale Erkrankungen bzw. Atemwegserkrankungen behandelt werden.

Insbesondere kann es sich bei der bronchopulmonalen Erkrankung um eine entzündliche oder nichtentzündliche, insbesondere entzündliche Erkrankung der oberen oder unteren Atemwege handeln.

Des weiteren kann es sich bei der bronchopulmonalen Erkrankung eine entzündliche, insbesondere infektexazerbierte und/oder steroidpflichtige Atemwegserkrankung handeln.

Beispielsweise kann es sich bei der bronchopulmonalen Erkrankung um Asthma bronchiale oder Bronchitis handeln.

Weiterhin kann die bronchopulmonale Erkrankung eine chronische obstruktive Lungenerkrankung (COPD) sein, insbesondere eine chronische obstruktive Bronchitis oder ein Lungenemphysem.

Des weiteren kann die bronchopulmonale Erkrankung eine tabakrauchinduzierte, insbesondere nikotininduzierte akute oder chronische Atemwegsentzündung sein.

Im Rahmen der erfindungsgemäßen Verwendung bzw. Kombinationstherapie können auch Frühformen von COPD, insbesondere im Stadium 0 oder I nach GOLD, oder auch Frühformen des Asthma bronchiale, insbesondere im Stadium 0 oder I nach GINA, behandelt werden.

Insbesondere können im Rahmen der erfindungsgemäßen Verwendung bzw. Kombinationstherapie Frühformen von COPD, insbesondere im Stadium 0 oder I nach GOLD, oder Frühformen des Asthma bronchiale, insbesondere im Stadium nach GINA, behandelt werden, und auf diese Weise kann eine Exazerbationsprophylaxe vor oder nach erfolgter Exazerbation bzw. eine Verhinderung oder Verlangsamung des Krankheitsprogresses vor oder nach erfolgter Exazerbation erreicht werden.

Des weiteren betrifft die vorliegende Erfindung die Verwendung mindestens eines systemisch applizierbaren, insbesondere peroralen Monoterpens, vorzugsweise 1,8-Cineol, insbesondere wie zuvor beschrieben, zur insbesondere synergistischen Wirkungssteigerung mindestens eines topisch applizierbaren, insbesondere inhalativen Atemwegstherapeutikums bei der prophylaktischen und/oder therapeutischen Behandlung von Atemwegserkrankungen, insbesondere bronchopulmonalen Erkrankungen.

Weiterhin betrifft die vorliegende Erfindung gemäß einem wiederum weiteren Aspekt der vorliegenden Erfindung die Verwendung mindestens eines systemisch applizierbaren, insbesondere peroralen Monoterpens, vorzugsweise 1,8-Cineol, insbesondere wie zuvor beschrieben, zur insbesondere synergistischen Steigerung der antiinflammatorischen und/oder antioxidativen Wirkung topischer, insbesondere inhalativer Kortikosteroide, insbesondere Glukokortikoide.

Gleichermaßen betrifft die vorliegende Erfindung gemäß einem wiederum weiteren Aspekt die Verwendung mindestens eines systemisch applizierbaren, insbesondere peroralen Monoterpens, vorzugsweise 1,8-Cineol, insbesondere wie zuvor beschrieben, zur Dosisreduktion von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

Weiterhin betrifft die vorliegende Erfindung gemäß einem anderen Aspekt der vorliegenden Erfindung die Verwendung mindestens eines systemisch applizierbaren, insbesondere peroralen Monoterpens, vorzugsweise 1,8-Cineol, insbesondere wie zuvor beschrieben, zur Induktion und/oder Verstärkung des steroidpermissiven Effektes von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

Ebenfalls betrifft die vorliegende Erfindung gemäß einem weiteren Aspekt die Verwendung mindestens eines systemisch applizierbaren, insbesondere peroralen Monoterpens, vorzugsweise 1,8-Cineol, insbesondere wie zuvor beschrieben, zur Vermeidung oder Reduktion eines Gewöhnungseffektes von Betasympathomimetika bei der prophylaktischen und/oder therapeutischen Behandlung von Atemwegserkrankungen, insbesondere bronchopulmonalen Erkrankungen, insbesondere unter Dauertherapie bei allen Schweregraden von COPD und Asthma bronchiale.

Außerdem betrifft die vorliegende Erfindung gemäß einem weiteren Aspekt die Verwendung mindestens eines systemisch applizierbaren, insbesondere peroralen Monoterpens, vorzugsweise 1,8-Cineol, insbesondere wie zuvor beschrieben, in Kombination mit mindestens einem topisch applizierbaren, insbesondere inhalativen Atemwegstherapeutikum, insbesondere in Kombination mit einem inhalativen Kortikosteroid, zur Reduktion des Bedarfs oder zum Ersatz systemischer Kortikosteroide oder anderer antiinflammatorisch und/ oder immunsuppressiv wirkender systemischer Substanzen bei der prophylaktischen und/oder therapeutischen Behandlung von Atemwegserkrankungen, insbesondere bronchopulmonalen Erkrankungen.

Des weiteren betrifft die vorliegende Erfindung gemäß einem wiederum weiteren Aspekt der vorliegenden Erfindung die Verwendung mindestens eines systemisch applizierbaren, insbesondere peroralen Monoterpens, vorzugsweise 1,8-Cineol, insbesondere wie zuvor beschrieben, in Kombination mit mindestens einem topisch applizierbaren, insbesondere inhalativen Atemwegstherapeutikum, insbesondere in Kombination mit einem inhalativen Kortikosteroid und gegebenenfalls weiter mit einem inhalativen Beta-2-Sympathomimetikum, zur Optimierung der Basistherapie bei Asthma bronchiale und COPD.

Im Rahmen der erfindungsgemäßen Verwendungen lassen sich auch systemische Organmitbeteiligungen bei allen schweren Formen von COPD behandeln.

Gleichermaßen dient die erfindungsgemäße Verwendung zur Modulation und Hemmung COPD-abhängiger und/oder COPD-unabhängiger Alterungsprozesse, insbesondere mit dem Ziel der Reduktion von Morbidität, der Erhöhung der Lebensqualität und/oder der Lebenserwartung ("Anti-Aging").

Weiterhin kann im Rahmen der erfindungsgemäßen Verwendung das Monoterpen, insbesondere 1,8-Cineol, als Induktor der NO-Produktion für die Behandlung der primären und sekundären pulmonal-arteriellen Hypertonie (PAH) bei COPD und Asthma bronchiale eingesetzt werden.

Des weiteren kann das Monoterpen, insbesondere 1,8-Cineol, im Rahmen der erfindungsgemäßen Verwendung zur Verbesserung der Gewebs- und/oder Mikroperfusion sowie der Bronchodilatation bei NO-Mangelsituationen eingesetzt werden.

Gleichermaßen kann im Rahmen der erfindungsgemäßen Verwendung das Monoterpen, insbesondere 1,8-Cineol, zur Induktion der NO-Produktion bei rezidivierenden Infekten der oberen und unteren Atemwege oder bei infektunabhängigen Exazerbationen, insbesondere durch Zigarettenrauchen und/oder Ozoneinfluß, oder zur Normalisierung noxenabhängiger oder noxenunabhängiger NO-Mangelsituationen eingesetzt werden.

Gleichermaßen kann im Rahmen der erfindungsgemäßen Verwendung das Monoterpen, insbesondere 1,8-Cineol, als Antioxidans und/oder NO-Induktor bei durch Zigarettenrauch induzierten Organschäden, insbesondere der Lunge, des Herzens, des Gehirns, der Nieren und des venösen und arteriellen Gefäßsystems, eingesetzt werden.

Gleichermaßen kann im Rahmen der erfindungsgemäßen Verwendung das Monoterpen, insbesondere 1,8-Cineol, zur kombinierten antiinflammatorischen und/oder antioxidativen Therapie der persistierenden und/oder progredienten Atemwegsentzündung nach Aufgabe des Rauchens, gegebenenfalls unter Co-Medikation mit anderen Atemwegstherapeutika, eingesetzt werden, insbesondere mit dem Ziel der Retardierung der Emphysementwicklung, der respiratorischen Insuffizienz und/oder der Entwicklung peripherer Atemwegsobstruktionen.

Gleichermaßen kann im Rahmen der erfindungsgemäßen Verwendung das Monoterpen, insbesondere 1,8-Cineol, zur Beeinflussung des gesamten Multiorganalterungsprozesses infolge antiinflammatorischer und/oder antioxidativer Wirkeigenschaften durch frühzeitige Langzeittherapie eingesetzt werden.

Weiterer Gegenstand der vorliegenden Erfindung gemäß einem wiederum weiteren Aspekt der vorliegenden Erfindung ist die Verwendung mindestens eines systemisch applizierbaren, insbesondere peroralen Monoterpens und mindestens eines topisch applizierbaren, insbesondere inhalativen Atemwegstherapeutikums als Kombinationstherapeutikum und/oder zu Zwecken der Co-Medikation zur prophylaktischen und/oder therapeutischen Behandlung von Atemwegserkrankungen, insbesondere bronchopulmonalen Erkrankungen.

Wiederum weiterer Gegenstand der vorliegenden Erfindung ist die Verwendung mindestens eines systemisch applizierbaren, insbesondere peroralen Monoterpens und mindestens eines topisch applizierbaren, insbesondere inhalativen Atemwegstherapeutikums zur Herstellung eines Kombinationstherapeutikums, insbesondere in Form eines Kit, zur prophylaktischen und/oder therapeutischen Behandlung von Atemwegserkrankungen, insbesondere bronchopulmonalen Erkrankungen.

Gleichermaßen ist Gegenstand der vorliegenden Erfindung auch ein Kombinationstherapeutikum, insbesondere in Form eines Kit, vorzugsweise zur prophylaktischen und/oder therapeutischen Behandlung Atemwegserkrankungen, insbesondere bronchopulmonalen Erkrankungen, wobei das Kombinationstherapeutikum mindestens ein systemisch applizierbares, insbesondere perorales Monoterpen einerseits und mindestens ein topisch applizierbares, insbesondere inhalatives Atemwegstherapeutikum andererseits umfaßt, insbesondere als räumlich getrennte Komponenten (sogenanntes "Kit-of-parts").

Weiterhin ist Gegenstand der vorliegenden Erfindung auch ein Verfahren zur Behandlung des menschlichen oder tierischen Körpers, insbesondere ein Verfahren zur prophylaktischen und/oder therapeutischen Behandlung von Atemwegserkrankungen, insbesondere bronchopulmonalen Erkrankungen, wobei mindestens ein Monoterpen und mindestens ein Atemwegstherapeutikum in jeweils wirksamen, insbesondere pharmazeutisch wirksamen Mengen eingesetzt werden, wobei das Monoterpen systemisch, insbesondere peroral appliziert wird und das Atemwegstherapeutikum topisch, insbesondere inhalativ appliziert wird.

Wiederum weiterer Gegenstand der vorliegenden Erfindung ist die Verwendung mindestens eines systemisch applizierbaren bzw. applizierten, insbesondere peroralen Monoterpens, vorzugsweise 1,8-Cineol, zur prophylaktischen und/oder kurativen Behandlung von Atemwegserkrankungen, insbesondere bronchopulmonalen Erkrankungen, bei Rauchern und/oder Exrauchern (d. h. also bei aktiven wie passiven Rauchern) bzw. zur Herstellung eines Arzneimittels zur prophylaktischen und/oder kurativen Behandlung von Atemwegserkrankungen, insbesondere bronchopulmonalen Erkrankungen, bei Rauchern oder Exrauchern. Dabei kann das Monoterpen gegebenenfalls zusammen mit mindestens einem topisch applizierbaren, insbesondere inhalativen Atemwegstherapeutikum, insbesondere als Kombinationstherapie und/oder in Co-Medikation, eingesetzt werden. Für weitergehende diesbezügliche Einzelheiten kann auf die vorstehenden Ausführungen zu den anderen Erfindungsaspekten verwiesen werden.

Gleichermaßen Gegenstand der vorliegenden Erfindung ist die Verwendung mindestens eines systemisch applizierbaren bzw. applizierten, insbesondere peroralen Monoterpens, vorzugsweise 1,8-Cineol, zur prophylaktischen und/oder kurativen Behandlung antioxidativer und/oder antiinflammatorischer Prozesse im menschlichen Körper insbesondere von Rauchern bzw. zur Herstellung eines Arzneimittels zur prophylaktischen und/oder kurativen Behandlung antioxidativer und/oder antiinflammatorischer Prozesse im menschlichen Körper insbesondere von Rauchern. Dabei kann das Monoterpen zusammen mit mindestens einem topisch applizierbaren, insbesondere inhalativen Atemwegstherapeutikum, insbesondere als Kombinationstherapie und/oder in Co-Medikation, eingesetzt werden. Für weitergehende Einzelheiten zu diesem Erfindungsaspekt kann Bezug genommen werden auf die vorstehenden Ausführungen zu den übrigen Erfindungsaspekten, welche diesbezüglich gleichermaßen entsprechend gelten.

Schließlich ist Gegenstand der vorliegenden Erfindung auch die Verwendung mindestens eines systemisch applizierbaren, insbesondere peroralen Monoterpens, vorzugsweise 1,8-Cineol, zur Erhöhung der Kortikosteroidsensitivität von Rauchern, insbesondere bei der prophylaktischen und/oder therapeutischen Behandlung von Atemwegserkrankungen, insbesondere bronchopulmonalen Erkrankungen. Dabei kann das Monoterpen gegebenenfalls zusammen mit mindestens einem topisch applizierbaren, insbesondere inhalativen Atemwegstherapeutikum, insbesondere als Kombinationstherapie und/oder in Co-Medikation, eingesetzt werden kann. Für weitergehende Einzelheiten zu diesem Erfindungsaspekt kann auf die vorstehenden Ausführungen zu den übrigen Erfindungsaspekten Bezug genommen werden.

Speziell bei der Behandlung von Rauchern mit einem systemischen bzw. peroralen Monoterpen, vorzugsweise 1,8-Cineol, läßt sich die nikotininduzierte Wirkung kontrollieren: Sauerstoffradikale, wie sie infolge des Rauchens gebildet werden, reduzieren nämlich die Kortikosteroidempfindlichkeit, welche charakteristisch niedrig beispielsweise für die COPD ist. Eine Zusatztherapie bei Rauchern, insbesondere mit bronchopulmonalen Erkrankungen, wie COPD, mit einem Monoterpen, vorzugsweise 1,8-Cineol, oder aber eine Therapie bei Rauchern oder Exrauchern ohne Atemwegserkrankungen mit 1,8-Cineol wirkt der Entwicklung von bronchopulmonalen Erkrankungen, wie z. B. COPD, überraschenderweise präventiv entgegen bzw. reduziert oder vermindert im Falle bestehender Atemwegserkrankungen Exazerbationen und einen progredienten Verlauf. Weiterhin ist speziell 1,8-Cineol speziell auch zum Schutz gegen schädliche Umwelteinflüsse geeignet.

Durch die systemische Gabe des Monoterpens, vorzugsweise 1,8-Cineol, läßt sich - wie nachfolgend noch ausgeführt - die Lungenfunktion signifikant verbessern, insbesondere im Rahmen einer Kombinationstherapie mit inhalativen bzw. topischen Atemwegstherapeutika, was unter anderem auf die antiinflammatorische und/oder antioxidative Wirkung des Monoterpens, vorzugsweise 1,8-Cineol, zurückzuführen ist. Das systemische Monoterpen, vorzugsweise 1,8-Cineol, hemmt die prooxidativen Wirkungen topischer Kortikosteroide und vermittelt selbst eine nichtsteroidartige antioxidative Wirkung. Dadurch wird die gewünschte Steroidsensivität erhöht, insbesondere auch in Fällen reduzierter Steroidempfindlichkeit bei COPD. Aufgrund der Exposition der Atemwege zu inhalativen Noxen, insbesondere Zigarettenrauch, beziehen sich die positiven Effekte speziell auf inhalative Steroide.

Es versteht sich von selbst, daß in bezug auf weitergehende Einzelheiten zu den einzelnen Erfindungsaspekten zu den ausführlichen Ausführungen im Zusammenhang mit dem ersten Erfindungsaspekt Bezug genommen werden kann, welche für die anderen Erfindungsaspekte entsprechend gelten.

Im Rahmen der vorliegenden Erfindung wurde insbesondere die synergistische antiinflammatorische Wirkung von Monoterpenen und topischen Atemwegstherpaeutika (z. B. inhalativen Glukokortikoiden) gefunden.

Es wurde insbesondere überraschend als Grundlage der vorliegenden Erfindung gefunden, daß therapeutisch relevante Konzentrationen von 1,8-Cineol die antiinflammatorische Wirkung topischer Atemwegstherapeutika, insbesondere topischer Glukokortikoide, durch eine synergistische Hemmung der Zytokinproduktion signifikant verstärken. Die Anmelderin konnte Nachweise einer synergistischen antiinflammatorischen Wirkung von Monoterpenen und topischen Atemwegstherapeutika, insbesondere topischen Glukokortikoiden, erbringen. So hemmen beispielsweise selbst niedrige therapeutische Konzentrationen von 1,8-Cineol (z. B. 10⁻⁶ mol/l) die Produktion von IL-1beta signifikant im Vergleich zu subtherapeutischen Konzentrationen von Beclometason, für das in alleiniger Verwendung keine signifikante Hemmung nachgewiesen werden konnte. Dagegen konnte durch Kombination von 1,8-Cineol und inhalativem Glukokortikoid (Beclometason) eine signifikante Hemmung auch für subtherapeutische und therapeutische Konzentrationen von Beclometason mit einer resultierenden Intensivierung der Wirkung von ICS nachgewiesen werden. Weitergehende klinische Studien zeigen die Möglichkeit der Reduktion der Dosismenge der inhalativen ICS von bis zu 30 % bis 50 % und mehr unter systemischer Langzeittherapie mit 1,8-Cineol (z. B. Soledum^{®}-Kapseln).

Die wesentliche klinische Bedeutung ist die Intensivierung der antiinflammatorischen Wirkung kleinster, in der Peripherie der Lunge abnehmender Konzentrationen gebräuchlicher ICS (Dosieraerosole und Pulverpräparate). Der Einsatz der erfindungsgemäßen Kombination aus systemischem Monoterpen und topischem Atemwegstherapeutikum, insbesondere topischem Glukokortikoid, (z. B. perorales 1,8-Cineol und ICS bzw. Beclometason) ist insbesondere zur Therapie der peripher ablaufenden Atemwegsentzündung bei Asthma bronchiale und COPD als neues Therapiekonzept und zur Beeinflussung der steroidrefraktären Progression der Lungenerkrankung von Vorteil, um der Entwicklung der irreversiblen respiratorischen Insuffizienz vorzubeugen. Von besonderem klinischem Wert aber ist der Therapieeinsatz von Monoterpenen, insbesondere 1,8-Cineol, zur Prophylaxe und Therapie der Frühformen der COPD (GOLD 0 oder GOLD I), für die bisher keine antiinflammatorische Therapie von allen Lungengesellschaften weltweit empfohlen wird.

Des weiteren wird durch eine kombinierte Therapie von systemischem Monoterpen und topischem Atemwegstherapeutikum, insbesondere topischem Glukokortikoid, (z. B. perorales 1,8-Cineol und ICS bzw. Beclometason) der steroidrefraktären Atemwegsentzündung durch Zigarettenrauch und auch andere schädliche, proinflammatorische oder oxidativ wirksame Umweltstoffe, wie insbesondere Ozon (O₃), und der Entwicklung einer COPD prophylaktisch und/oder therapeutisch entgegengewirkt.

Ein weiterer wesentlicher Aspekt der vorliegenden Erfindung ist insbesondere auch die prophylaktische und abschwächende Wirkung auf Entzündungen durch Monoterpene, insbesondere 1,8-Cineol, in Kombination mit topischen Atemwegstherapeutika, insbesondere topischen Glukokortikoiden, oder auch durch Monoterpene allein, insbesondere 1,8-Cineol, bei Rauchern zur Verhinderung und Besserung der durch Zigarettenrauch vermittelten Atemwegsschäden. Diese treten insbesondere noch viele Jahre nach Aufgabe des Rauchens oder der Einwirkung anderer Noxen auf und sind klinisch gekennzeichnet durch eine progrediente Obstruktion und Emphysementwicklung mit respiratorischer Insuffizienz unter laufender antiobstruktiver Therapie.

Speziell für das Monoterpen 1,8-Cineol als Zusatztherapie zu modernen kombinierten Therapieformen (z. B. ICS plus LABA oder ICS in Kombination mit Vagolytika oder aber ICS oder SABA allein) wurde unter Langzeittherapie eine Hemmung der Produktion von TNF-α und eine vermehrte Produktion von IL-8 in ex-vivo stimulierten peripheren humanen Monozyten bei Exrauchern im Vergleich zu normalen Monozyten von Nichtrauchern überraschend gefunden, die bereits vor mehr als zehn Jahren das Rauchen aufgegeben hatten. Monozyten wurden von diesen Patienten unter laufender Therapie mit ICS gewonnen und mit einem topischen Glukokortikoid in vitro dosisabhängig inkubiert. Die Ergebnisse zeigen insbesondere im Vergleich zu einem normalen Probandenkollektiv ohne Therapie mit 1,8-Cineol eine stärkere und früher einsetzende Hemmwirkung auf die Produktion von IL-8 und TNF-α in Monozyten von Patienten mit COPD. Diese Ergebnisse führen zu der neuen Erkenntnis, daß die alleinige Hemmung steroidsensitiver Mechanismen den Krankheitsverlauf nicht ausreichend beeinflussen kann, so daß eine systemische Zusatztherapie mit Monoterpenen die persistierende Atemwegsentzündung bei COPD entscheidend verbessert, bei persistierender Entzündung Exazerbationen reduzieren hilft und gerade in der Peripherie die Wirkung kleinerer, atemwegsrelevanter Konzentrationen topischer Glukokortikoide oder anderer inhalativer Atemwegstherapeutika intensiviert.

Die Hemmung von Exazerbationen gilt derzeit als eines der bedeutendsten Therapieziele bei Rauchern und Exrauchern mit COPD, die durch nichtsteroidale Effekte des Monoterpens 1,8-Cineol gebessert werden können. Zusätzlich steigert 1,8-Cineol antiinflammatorische und antiobstruktive Effekte von topischen Atemwegstherapeutika, insbesondere topischen Glukokortikoiden, so daß auch bisher noch nicht bekannte pharmazeutische Kombinationen aus z. B. ICS und 1,8-Cineol oder einem anderen Monoterpen, LABA und 1,8-Cineol, SABA und 1,8-Cineol sowie von ICS, SABA und 1,8-Cineol oder von ICS, LABA und 1,8-Cineol als eine therapeutische Alternative zur Therapie von Asthma und COPD in allen Schweregraden und zur zusätzlichen Behandlung der aktiven systemischen Komponente bei COPD durch die systemische Verfügbarkeit von Monoterpenen, insbesondere in dünndarmlöslichen Kapseln oder als Pulver, geeignet sind.

Auf der Suche nach der zugrundeliegenden Eigenschaft von Monoterpenen, insbesondere 1,8-Cineol, zur Intensivierung antiinflammatorischer Wirkungen topischer Steroide wurde im Rahmen der vorliegenden Erfindung zudem eine antioxidative Wirkung für 1,8-Cineol durch Hemmung der Produktion von Superoxiden (O₂⁻-Radikale), der Aktivität von Superoxiddismutasen (SOD) und von Wasserstoffperoxid (H₂O₂) gefunden, das als Endprodukt der Oxidation die Produktion von Entzündungsmediatoren, insbesondere von Zytokinen und Arachidonsäuremetaboliten, stimuliert. Hierbei wurde erstmalig eine Hemmung der Spontanproduktion von O₂⁻-Radikalen bei therapeutischen Konzentrationen von 1,8-Cineol nachgewiesen, das bei niedrigeren, atemluftrelevanten Konzentrationen O₂⁻-Radikale stimuliert und im therapeutischen Bereich die Produktion von O₂⁻-Radikalen und H₂O₂ hemmt. Als Ursache für diese antioxidativen Wirkungen von 1,8-Cineol wurde überraschenderweise 1,8-Cineol als aktiver Induktor der NO- Produktion gefunden, das über diesen Mechanismus O₂⁻ dem Organismus als Substrat zur Bildung der NO-Produktion entzieht. Somit wurde gefunden, daß Monoterpene, wie 1,8-Cineol, aktiv die NO-Produktion durch Vermittlung antioxidativer Wirkungen induzieren. Hierbei konnten erstmalig modulierende Einflüsse von 1,8-Cineol zur Kontrolle oxidativer, zellschädigender und proinflammatorischer Einflüsse durch Hemmung von O₂⁻-Radikalen und einer gegenläufigen Stimulation von antiinflammatorischem und vasodilatierendem NO im therapeutischen Bereich von 1,8-Cineol nachgewiesen werden. Diese Ergebnisse sind von integraler Bedeutung für die Prophylaxe und Therapie, insbesondere von mit Zigarettenrauchen assoziierten Erkrankungen der Lunge, einschließlich des Lungenemphysems und der Regulation des Tonus pulmonaler Gefäße, sowie von Schäden des großen und kleinen Kreislaufs. So wird eine erhöhte Produktion von O₂⁻-Radikalen durch Zigarettenrauch, Infekte, Nanopartikel, Ozon, Allergene und weitere Umwelteinflüsse vermittelt, die durch Langzeittherapie mit 1,8-Cineol dauerhaft gehemmt und außerdem vorteilhaft als Substrat für die Produktion von NO genutzt werden kann.

NO ist bekannt als antiinflammatorischer Mediator, Vasodilatator, Hemmstoff von Entzündungsmediatoren, Histamin, der Granulozytenadhäsion und der Thrombozytenaggregation sowie als Aktivator der ziliären Funktion und mukosalen Clearance und schützt zusammenfassend vor respiratorischen Infekten und Exazerbationen von Asthma und COPD in allen Erkrankungsstadien. In dieser Hinsicht ist 1,8-Cineol geeignet als Dauertherapeutikum, das die bei chronischer Bronchitis, COPD, Emphysem und Rhinosinusitis supprimierte NO-Produktion durch günstigen Abbau von O₂⁻-Radikalen mit Induktion von NO normalisiert und den jeweiligen Erfordernissen entsprechend durch Modulation adäquat anpaßt. Daraus ergeben sich insbesondere neue Indikationen für den Einsatz von Monoterpenen, insbesondere 1,8-Cineol, bevorzugt in einer höheren, systemisch wirksamen Tagesdosis von z. B. 600 bis 1.200 mg, zur Regulation der Organperfusion und dem Schutz oberer und unterer Atemwege, einschließlich der Lunge, vor pathogen wirksamen Noxen, insbesondere dem Zigarettenrauchen, respiratorischen Infekten und der allergischen und nichtallergischen Atemwegsentzündung bei Hyperreaktivität, Asthma und Rhinitis.

Zusammenfassend führen die im Rahmen der vorliegenden Erfindung aufgefundenen Erkenntnisse von Monoterpenen zu einem völlig neuen Verständnis der vom Fachmann bislang nicht anerkannten Gruppe der sogenannten Sekretolytika und Mukolytika unter Einbezug natürlicher ätherischer Öle oder Mischterpenen, Ambroxol und N-Acetylcystein, deren Einsatz bisher primär der Schleimlösung, aber nicht kausal der Freisetzung von NO oder der Beeinflussung anderer Mechanismen und somit nicht primär der Prophylaxe und Therapie der multifaktoriellen Atemwegsentzündung diente. Dies bedeutet, daß die herkömmliche, zeitlich begrenzte Therapie mit zumeist nicht effektiven Substanzen nur die eigentliche Mukus-Hypersekretion trifft und daher eine Langzeittherapie zur Verhinderung der Entwicklung und Progression der Atemwegsentzündung bei COPD und Asthma durch frühzeitigen Einsatz von effektiven Substanzen mit kombiniertem antioxidativem und antiinflammatorischem Wirkprofil und einem nichtsteroidalen antiinflammatorischen Wirkmechanismus zur Verbesserung der Wirkung von topischen Atemwegstherapeutika, insbesondere Glukokortikoiden, sowie einer guten Verträglichkeit ohne Steroidnebenwirkungen entgegen den allgemeingültigen Richtlinien nationaler und internationaler Lungengesellschaften dringlich vorzuschlagen ist. Hierbei dürften effektive Substanzen, wie Monoterpene, insbesondere 1,8-Cineol, zukünftig eine zentrale Rolle spielen aufgrund ihrer besseren Verfügbarkeit in den Atemwegen durch die hohe Lipophilie und die Exhalation des Wirkstoffs nach alveolarer Aufnahme in die Lungenperipherie aus der Blutbahn. Für Monoterpene, insbesondere 1,8-Cineol, ist daher eine neue Klassifikation mit Zugehörigkeit zu einer neuen Substanzgruppe als "Non-Steroidal Airway Inflammation Modifier (NSAIM)" vorzuschlagen.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt. Die folgenden Ausführungsbeispiele dienen lediglich der Veranschaulichung der vorliegenden Erfindung, ohne sie jedoch hierauf zu beschränken.

### Ausführungsbeispiele:

### Beispiel 1: In-vitro-Untersuchungen

Durch in-vitro-Untersuchungen wurde gefunden, daß Monoterpene (hier konkret: 1,8-Cineol) die Wirkung von inhalativen Atemwegstherapeutika, insbesondere Glukokortikoiden (hier konkret: Beclometason), in signifikanter, insbesondere synergistischer Weise zu steigern imstande ist.

Es wurde überraschend gefunden, daß therapeutisch relevante Konzentrationen von 1,8-Cineol die antiinflammatorische Wirkung topischer Glukokortikoide durch eine synergistische Hemmung der Zytokinproduktion signifikant verstärken (siehe Tabelle 1).

Die Tabelle 1 zeigt die synergistische Aktivität von 1,8-Cineol (10⁻⁶ mol/l) und Beclometason auf die LPS-stimulierte Produktion von IL-1beta in humanen Monozyten in vitro. Die monozytäre IL-lbeta Produktion (n = 14-15, 4 Experimente) in Monozyten wird durch Cineol (10⁻⁶ mol/l) im Vergleich zur Kontrolle signifikant gehemmt. Cineol und Beclometason hemmen synergistisch die IL-1beta-Produktion stärker als Beclometason allein. Im Vergleich zu Cineol (10⁻⁶ mol/l) allein wird die IL-1 beta-Produktion durch Zusatz von Beclometason auch synergistisch und signifikant stärker gehemmt (p < 0.05).

**Tabelle 1**

| **Synergistische Aktivität von 1,8-Cineol (10⁻⁶ mol/l) und Beclometason ("Becl.") auf die LPS-stimulierte Produktion von IL-1beta in humanen Monozyten in vitro** | | | | | |
|---|---|---|---|---|---|
| | **IL-1beta** | **Effekt vs. Kontrolle** | | **Effekt vs. Cineol 10⁻⁶ mol/l** | |
| mol/l | **pg/5 x 10⁴ Zellen** | **Hemmung (%)** | **p-Wert** | **Hemmung (%)** | **p-Wert** |
| Kontrolle | **5252 ± 1017** | **0 ± 19** | - | - | - |
| Cineol 10⁻⁶ | **3548 ± 600** | **32.4 ± 17** | **0.0100** | **0 ± 17** | - |
| Beclometason 10⁻¹² | **5246 ± 1028** | **0.1 ± 19** | **0.9634** | (+**47.8 ± 19**) | **0.0100** |
| Beclometason 10⁻¹¹ | **4217 ± 864** | **19.7 ± 20** | **0.0596** | **(+18.9 ± 20**) | **0.5557** |
| Beclometason 10⁻¹⁰ | **4047 ± 940** | **22.9 ± 23** | **0.0661** | **(+14 ± 23**) | **0.6945** |
| Beclometason 10⁻⁹ | **2654 ± 545** | **49.4 ± 20** | **0.0449** | **25.2 ± 20** | **0.0790** |
| Cineol 10⁻⁶ + Becl. 10⁻¹² | **3966 ± 642** | **24.5 ± 16** | **0.1904** | **(+11.8 ± 16**) | **0.0401** |
| Cineol 10⁻⁶ + Becl. 10⁻¹¹ | **2632 ± 474** | **49.9 ± 18** | **0.0100** | **25.8 ± 18** | **0.0538** |
| Cineol 10⁻⁶ + Becl. 10⁻¹⁰ | **2696 ± 607** | **48.7 ± 22** | **0.0088** | **24 ± 22** | **0.0443** |
| Cineol 10⁻⁶ + Becl. 10⁻⁹ | **347 ± 56** | **93.4 ± 16** | **0.0049** | **90.2 ± 16** | **0.0287** |

| | | | | | |
|---|---|---|---|---|---|
| Die monozytäre IL-1beta-Produktion (n = 14-15, 4 Experimente) in Monozyten wird durch 1,8-Cineol (10⁻⁶ mol/l) im Vergleich zur Kontrolle signifikant gehemmt. 1,8-Cineol und Beclometason hemmen synergistisch die IL-1beta-Produktion stärker als Beclometason allein. Im Vergleich zu Cineol (10⁻⁶ mol/l) wird die IL-1beta-Produktion durch Zusatz von Beclometason auch synergistisch und signifikant stärker gehemmt (p < 0.05). | | | | | |

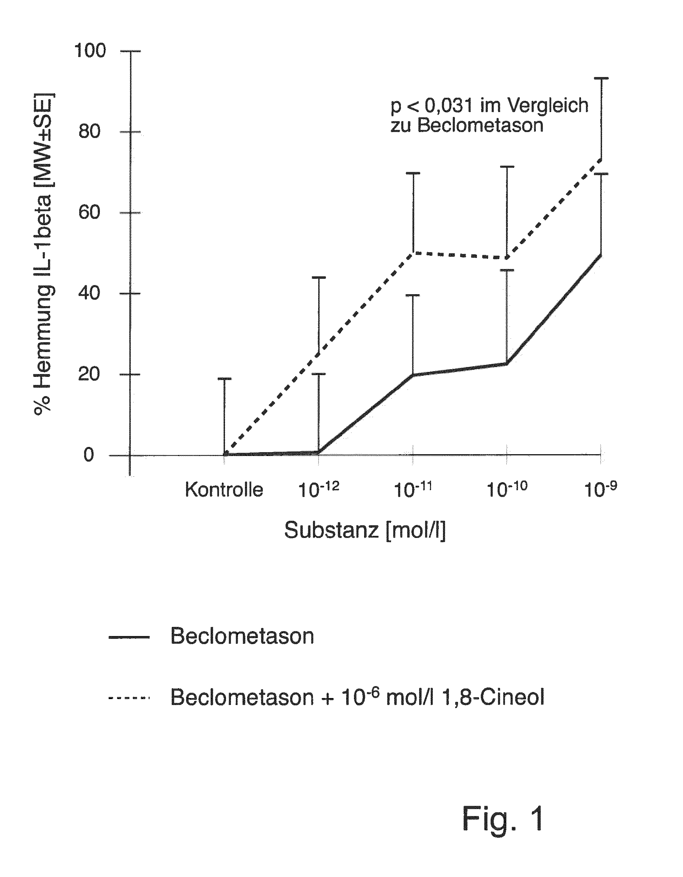

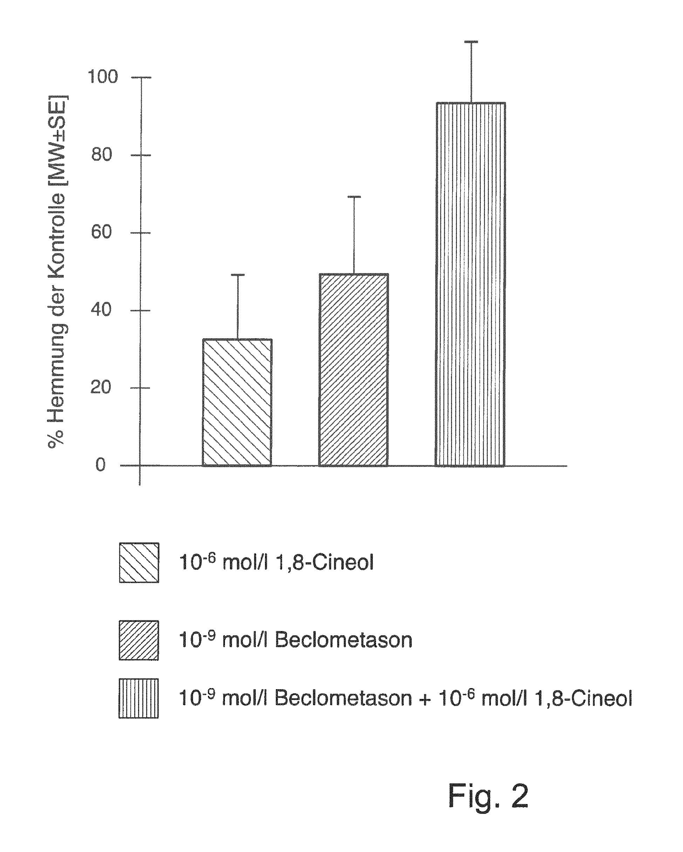

Die Ergebnisse aus humanen Monozytenkulturen zeigen erstmals, daß die LPS-stimulierte Produktion von IL-1beta durch Beclometason und 1,8-Cineol 10⁻⁶mol/l (73.4 ± 19 %) signifikant (p < 0.01) stärker gehemmt wird als durch Beclometason allein (49.9 ± 20 %) oder 1,8-Cineol allein (32.4 ± 17 %) (siehe Figs. 1 und 2). Dies ist der erste Nachweis einer synergistischen antiinflammatorischen Wirkung von systemischen Monoterpenen und topischen Glukokortikoiden.

In diesem Zusammenhang zeigt die Fig. 1 die Stimulierung der Wirkung des inhalativen Kortikosteroids Beclometason durch das Monoterpen 1,8-Cineol (10⁻⁶ mol/l) in LPS-stimulierten humanen Monozyten in vitro; die Zugabe bereits geringer Mengen von 1,8-Cineol bewirkt eine signifikante Steigerung der Wirkung des Beclometasons, einhergehend mit einer gesteigerten Hemmung von IL-1beta. Die Fig. 1 zeigt die synergistische Wirkung von 1,8-Cineol und Beclometason: Monozyten (n = 14-15, 4 Experimente) von gesunden Probanden (10⁵/ml) wurden über 20 Stunden mit 1,8-Cineol (10⁻⁶mol/l = 0.015 µg/ml) und Beclometason (10⁻¹²- 10⁻⁹ mol/l) in Gegenwart von LPS (10 mg/ml) inkubiert. In Zellkulturüberständen wurde die Produktion von IL-1 beta durch ELISA (Fa. Cayman, AnnArbor, MI, USA) bestimmt. 1,8-Cineol allein (-32 % ± 17 %, p = 0.01) und therapeutisch relevante Konzentrationen von Beclometason (10⁻⁹ mol/l, p = 0.045) hemmen die Produktion von IL-1beta. Dagegen wird die LPS-stimulierte Produktion durch eine Kombination von 1,8-Cineol (10⁻⁶ mol/l) plus Beclometason (10⁻¹¹ - 10⁻⁹mol/l) signifikant stärker gehemmt (p < 0.031) als durch Beclometason allein. 1,8-Cineol intensiviert insbesondere die Wirkung subtherapeutischer Konzentrationen von Beclometason, die für periphere Atemwege relevant sind.

Die Fig. 2 zeigt die Effekte von 1,8-Cineol auf die IL-1beta-Hemmung durch Beclometason in vitro. Die Fig. 2 veranschaulicht gleichermaßen die synergistische Wirkung von 1,8-Cineol und Beclometason bei höheren therapeutischen Konzentrationen: Die LPS-stimulierte Produktion von IL1-beta (n=14-15, 4 Experimente) wird durch therapeutische relevante Konzentrationen von 1,8-Cineol dosisabhängig und signifikant (p<0.01) im Vergleich zur LPS-Kontrolle gehemmt. Die Wirkung therapeutisch relevanter Konzentrationen von Beclometason wird durch 1,8-Cineol im Vergleich zu Beclometason plus 1,8-Cineol signifikant (p < 0.01) stärker gehemmt. Um diese Wirkung zu erreichen, müssen wenigstens 10fach höhere Konzentrationen von beiden Substanzen allein vorliegen.

Des weiteren lassen die Ergebnisse erstmalig nachweisen, daß selbst niedrige therapeutische Konzentrationen von 1,8-Cineol (10⁻⁶ mol/l) die Produktion von IL-1beta im Vergleich zu subtherapeutischen Konzentrationen von Beclometason (Beclometason: 10⁻¹² mol/l: 0 %; 10⁻¹¹ mol/l: - -19.7 % ± 20 %; 10⁻¹⁰ mol/l: -22.9 % ± 13 %), für das keine signifikante Hemmung nachgewiesen wurde, signifikant hemmen. Dagegen konnte durch Kombination von 1,8-Cineol und Beclometason eine signifikante Hemmung auch für subtherapeutische und therapeutische Konzentrationen von Beclometason mit einer resultierenden Intensivierung der Wirkung von ICS nachgewiesen werden.

Diese Befunde bestätigen die nachfolgend noch geschilderten klinischen Daten in bezug auf eine signifikante Reduktion der inhalativen ICS-Dosis von bis zu 60 % unter Langzeittherapie mit peroralem 1,8-Cineol (Soledum^{®}-Kapseln).

Ein weiterer wesentlich neuer Aspekt der vorliegenden Daten ist die prophylaktische und abschwächende Wirkung auf Entzündungen durch 1,8-Cineol in Kombination mit topischen Glukokortikosteroiden oder auch durch 1,8-Cineol allein bei Rauchern zur Verhinderung und Besserung der durch Zigarettenrauch vermittelten Atemwegsschäden. Diese treten insbesondere noch viele Jahre nach Aufgabe des Rauchens oder der Einwirkung anderer Noxen auf und sind klinisch gekennzeichnet durch eine progrediente Obstruktion und Emphysementwicklung mit respiratorischer Insuffizienz unter laufender antiobstruktiver Therapie. Bezüglich der empfohlenen kombinierten Standardtherapieverfahren aus langwirksamen Beta-2-Sympathomimetika (LABA), kurzwirksamen Beta-2-Sympathomimetika (SABA) und inhalativen Steroiden (ICS) ergeben sich neue, bisher nicht bekannte Optionen zur Zusatztherapie mit 1,8-Cineol mit dem Ziel, topische Therapieeffekte zu verstärken und eine jetzt als Systemerkrankung identifizierte Atemwegserkrankung kombiniert topisch und systemisch mit zusätzlicher Intensivierung der topischen antiinflammatorischen und bronchodilatatorischen Therapie als Systemerkrankung durch Langzeittherapie zu behandeln.

Die Hemmung von Exazerbationen gilt derzeit als eines der wichtigsten Therapieziele bei Rauchern und Exrauchern mit COPD, die durch nichtsteroidale Effekte des Monoterpens 1,8-Cineol gebessert werden können. Zusätzlich verbessert 1,8-Cineol antiinflammatorische und antiobstruktive Effekte von ICS plus LABA, so daß auch bisher noch nicht bekannte pharmazeutische Kombinationsprodukte bzw. -kits, bestehend z. B. aus ICS + 1,8-Cineol oder einem anderen Monoterpen, LABA + 1,8-Cineol, SABA + 1,8-Cineol oder aber ICS + SABA + 1,8-Cineol oder ICS + LABA +1,8-Cineol, sowie moderne Kombinationen mit langwirksamen Vagolytika zukünftig als eine therapeutische Alternative zur Therapie von Asthma und COPD in allen Schweregraden und zur zusätzlichen Behandlung der aktiven systemischen Komponente bei COPD durch die systemische Verfügbarkeit von Monoterpenen in Form von dünndarmlöslichen Kapseln oder als Pulver geeignet sein werden.

Im Rahmen der vorliegenden Erfindung konnten erstmals modulierende antioxidative und antiinflammatorische Wirkungen von Monoterpenen (1,8-Cineol) zur Kontrolle oxidativer Prozesse und Induktion der Stickoxidproduktion (NO-Produktion) nachgewiesen werden. Auf der Suche nach der zugrundeliegenden Eigenschaft von Monoterpenen, insbesondere 1,8-Cineol, zur Intensivierung antiinflammatorischer Wirkungen topischer Steroide wurde eine antioxidative Wirkung für 1,8-Cineol durch Hemmung der Produktion von Superoxiden (O₂⁻-Radikale), der Aktivität von Superoxiddismutasen (SOD) und von Wasserstoffperoxid (H₂O₂) gefunden. Hierbei wurde erstmalig eine Hemmung der spontanen und stimulierten Produktion von Superoxiden (O₂⁻-Radikalen) bei therapeutischen Konzentrationen von 1,8-Cineol nachgewiesen, so daß die Bereitstellung des Substrats für die Dismutierung von O₂⁻-Radikalen über eine durch 1,8-Cineol partiell gehemmte Superoxid-Dismutaseaktivität auch bei niedrigeren, atemluftrelevanten Konzentrationen und im therapeutischen Bereich die Produktion von O₂⁻-Radikalen und H₂O₂ hemmte. Als wesentliche Ursache für diese antioxidativen Wirkungen von 1,8-Cineol wurde überraschenderweise 1,8-Cineol als aktiver Induktor der NO-Produktion gefunden, das über diesen zusätzlichen Mechanismus O₂⁻ dem Organismus als Substrat zur Bildung von NO-Produktion entzieht. Somit wurde gefunden, daß Monoterpene, wie 1,8-Cineol, aktiv die NO-Produktion durch Vermittlung antioxidativer Wirkungen induzieren (siehe Tabelle 2).

Die Tabelle 2 zeigt den Effekt von 1,8-Cineol auf die PMA-stimulierte Superoxid-Produktion (O₂⁻-Produktion) (in RPMI 1640) normaler humaner Monozyten in vitro. Die dosisabhängigen Effekte von 1,8-Cineol (4 Experimente, n = 9-14) auf die O₂⁻-Produktion wurden durch Bestimmung von INT-Formazan in Kulturüberständen (RPMI 1640) humaner Monozyten (10⁵/ml) nach 20 Stunden gemessen. Die O₂⁻-Produktion wurde nicht durch PMA (500 mmol/l) stimuliert. Für die statistische Analyse wurde der nichtparametrische Mann & Whitney-Test herangezogen (p < 0.05).

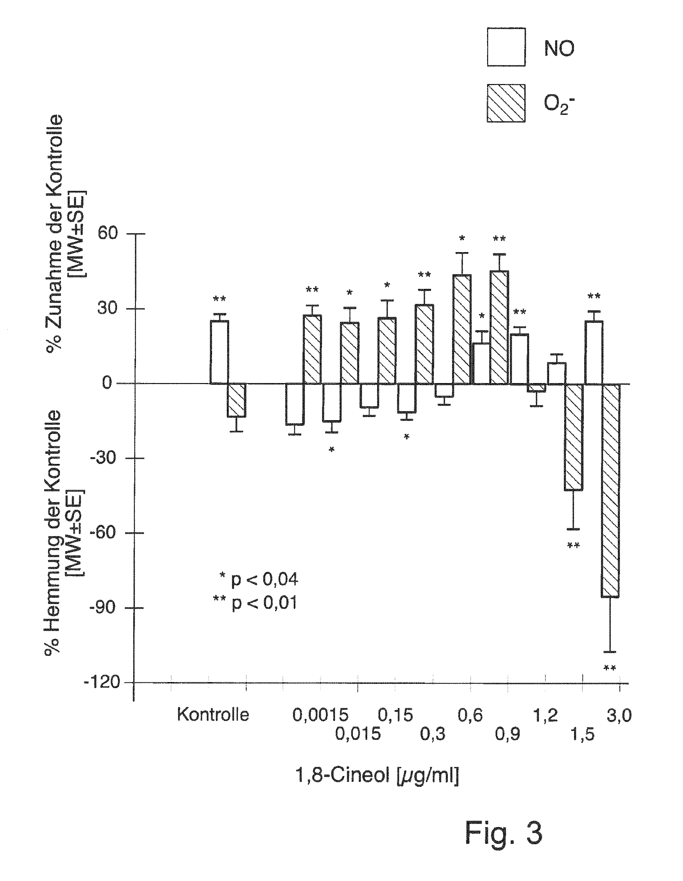

Hierbei konnten erstmalig modulierende Einflüsse von 1,8-Cineol zur Kontrolle oxidativer, zellschädigender und proinflammatorischer Einflüsse durch Hemmung von O₂⁻-Radikalen und einer gegenläufigen Stimulation von antiinflammatorisch und vasodilatierendem NO im therapeutischen Bereich von 1,8-Cineol nachgewiesen werden (siehe Fig. 3).

**Tabelle 2: Effekt von 1,8-Cineol auf die PMA-stimulierte Superoxidproduktion (O₂⁻-Produktion) in RPMI 1640 normaler humaner Monozyten in vitro**

| **1,8-Cineol** | **INT-Formazan** | **Vergleich zur Kontrolle** | **p-Wert** |
|---|---|---|---|
| **µg/ml (mol/l)** | **(nmol/10⁵)** | **(%)** | |
| **spontan** | **4828 ± 251** | - | - |
| **PMA** | **4203 ± 267** | **-12.9 ± 6** | **0.0990** |
| **0.0015 (10⁻⁸)** | **5.364 ± 229** | **+27.6 ± 4** | **0.0083** |
| **0.015 (10⁻⁷)** | **5235 ± 341** | **+24.5 ± 6** | **0.0327** |
| **0.15 (10⁻⁶)** | **5327 ± 360** | **+26.7 ± 7** | **0.0378** |
| **0.3 (2 x 10⁻⁶)** | **5540 ± 359** | **+31.8 ± 6** | **0.0091** |
| **0.6 (4 x 10⁻⁶)** | **6045 ± 534** | **+43.8 ± 9** | **0.0277** |
| **0.9 (6 x 10⁻⁶)** | **6107 ± 437** | **+45.3 ± 7** | **0.0050** |
| **1.2 (8 x 10⁻⁶)** | **4099 ± 234** | **-2.5 ± 6** | **0.7721** |
| **1.5 (10⁻⁵)** | **2438 ± 389** | -**42 ± 16** | **0.0024** |
| **3 (2 x 10⁻⁵)** | **634 ± 139** | **-84.9 ± 22** | < **0.0001** |

Fig. 3 zeigt die konzentrationsabhängigen modulierenden Effekte von 1,8-Cineol auf die O₂⁻- und NO-Produktion in stimulierten humanen Monozyten in vitro: Nach Stimulation (20 Stunden) normaler humaner Monozyten (10⁵/ml) wird in der Kontrolle (d. h. ohne 1,8-Cineol) die Produktion von NO induziert und von O₂⁻ supprimiert. Niedrige Konzentrationen von 1,8-Cineol dagegen induzieren leichtgradig die O₂⁻-Produktion und hemmen bei subtherapeutischen Konzentrationen (0.15 µg/ml = 10⁻⁶ mol/l) die NO-Produktion. Im therapeutischen Bereich von 1,8-Cineol wird die O₂⁻-Produktion stark gehemmt in Gegenwart stimulierender Effekte auf die NO-Produktion (*p < 0.04, **p < 0.01).

Diese Ergebnisse sind von integraler Bedeutung für die Prophylaxe und Therapie insbesondere von mit Zigarettenrauchen assoziierten Erkrankungen der Lunge, einschließlich des Lungenemphysems und der Regulation des Tonus pulmonaler Gefäße, sowie von Schäden des großen und kleinen Kreislaufs. So wird eine erhöhte Produktion von O₂⁻-Radikalen durch Zigarettenrauch, Infekte, Nanopartikel, Ozon, Allergene und weitere Umwelteinflüsse vermittelt, die durch Langzeittherapie mit 1,8-Cineol dauerhaft gehemmt und außerdem vorteilhaft als Substrat für die Produktion von NO genutzt wird. NO ist bekannt als antiinflammatorischer Mediator, Vasodilatator, Hemmstoff von Entzündungsmediatoren, Histamin, der Granulozytenadhäsion und der Thrombozytenaggregation sowie als Aktivator der ziliären Funktion und mukosalen Clearance und schützt zusammenfassend vor respiratorischen Infekten und Exazerbationen von Asthma und COPD in allen Erkrankungsstadien. In dieser Hinsicht ist 1,8-Cineol geeignet als Dauertherapeutikum, das die bei chronischer Bronchitis, COPD, Emphysem und Rhinosinusitis supprimierte NO-Produktion durch günstigen Abbau von O₂⁻-Radikalen mit Induktion von NO normalisiert und den jeweiligen Erfordernissen entsprechend durch Modulation adäquat anpaßt. Daraus ergeben sich insbesondere neue Indikationen für den Einsatz von Monoterpenen, insbesondere 1,8-Cineol, bevorzugt in einer höheren, systemisch wirksamen Tagesdosis, zur Regulation der Organperfusion und dem Schutz oberer und unterer Atemwege, einschließlich der Lunge, vor pathogen wirksamen Noxen, insbesondere dem Zigarettenrauchen und anderen Emissionen oder Feinstäuben, respiratorischen Infekten und der allergischen und nichtallergischen Atemwegsentzündung bei Hyperreaktivität, Asthma und Rhinitis. Diese bisher unbekannten Eigenschaften zur Wirkung von Monoterpenen, insbesondere 1,8-Cineol, können dazu beitragen, künftig die Zusatztherapie mit 1,8-Cineol in der Literatur und den Therapieleitlinien erstmals zu etablieren.

### Beispiel 2: Klinische Ergebnisse

10 Patienten im Alter von 56 bis 72 Jahren mit persitierendem Asthma bronchiale (GINA II), welche mit einer Kombinationstherapie aus inhalativem Glukokortikoid (Beclometason, 2 x 200 µg/die inhalativ) und inhalativem langwirksamem Beta-2-Sympathomimetika (LABA, Salmeterol) sowie Theophyllin peroral behandelt wurden, erhielten eine Woche lang 1,8-Cineol (Soledum^{®}-Kapseln) 4 x 200 mg/die peroral. Bereits nach einwöchiger Therapie mit der vorgenannten Dosis konnte bei 8 der 10 Probanden eine leicht- bis mittelgradige Verbesserung der Lungenfunktion erreicht werden. Nach fortgesetzter zwölfwöchiger Dauertherapie hatte sich das persistierende Bronchialasthma derart stabilisiert, daß bei 7 der 10 Patienten der inhalative Glukokortikoidbedarf um bis zu 60 % reduziert werden konnte und bei 2 der 10 Patienten die inhalativen Glukokortikoide zeitweise sogar ganz abgesetzt werden konnten. Die Therapie wurde ohne Nebenwirkungen gut vertragen. Bei 5 der behandelten Patienten konnte auch der Betamimetikabedarf um bis zu 40 % reduziert werden.

Weitere 12 Patienten im Alter von 59 bis 78 Jahren mit persitierendem Asthma bronchiale (GINA III), welche mit einer Kombinationstherapie aus inhalativem Glukokortikoid (Beclometason, 2 x 400 µg/die inhalativ) und inhalativem langwirksamem Beta-2-Sympathomimetika (LABA, Salmeterol) sowie Theophyllin peroral behandelt wurden, erhielten eine Woche lang 1,8-Cineol (Soledum^{®}-Kapseln) 4 x 200 mg/die peroral. Bereits nach einwöchiger Therapie mit der vorgenannten Dosis konnte bei 9 der 12 Probanden eine leichtgradige Verbesserung der Lungenfunktion erreicht werden. Nach fortgesetzter zwölfwöchiger Dauertherapie hatte sich das persistierende Bonchialasthma derart stabilisiert, daß bei 9 der 12 Probanden der inhalative Glukokortikoidbedarf um bis zu 30 % reduziert werden konnte. Die Therapie wurde ohne Nebenwirkungen gut vertragen. Bei 4 der behandelten Patienten konnte auch der Betamimetikabedarf um bis zu 25 % reduziert werden.

Die Versuchsergebnisse belegen, daß Monoterpene die Wirksamkeit inhalativer Atemwegstherapeutika in signifikanter Weise steigern und auf diese Weise deren Dosisbedarf signifikant reduziert werden kann.

### Beispiel 3: Weitere klinische Ergebnisse

In einer placebokontrollierten Doppelbindstudie wurde die Wirkung einer Zusatztherapie von 1,8-Cineol in Form von dünndarmlöslichen Kapseln (Soledum^{®}-Kapseln, 3 x 200 mg/die, peroral) bei 242 Rauchern mit COPD (GOLD II bis III) auf die Exazerbationsrate und die Lungenfunktion in 3 Wintermonaten von zwei aufeinanderfolgenden Jahren untersucht. Beide Patientengruppen waren identisch bezüglich Alter, Geschlecht, Body-Mass-Index, Raucherstatus, Lungenfunktion und einer leitliniengerechten Medikation aus ICS, LA-BA, SABA, Anticholinergika und Theophyllin. Die Zusatztherapie mit 1,8-Cineol führte in der Verumgruppe im Vergleich zur Placebogruppe zu einer signifikanten Abnahme der Exazerbationsrate von -38.5 %. Zusätzlich wurden die Lungenfunktion (FEV1) signifikant in der Verumgruppe (+ 5.1 %) im Vergleich zur Placebogruppe (-1 %) gebessert. Auch klinische Parameter, wie der St. George's Respiratory Questionnaire (SGRQ), wurden in der Verumgruppe (-10.4 Einheiten) signifikant stärker als in der Placebogruppe (-5 Einheiten) gebessert. Die Ergebnisse zeigen somit erstmals, daß eine Zusatztherapie mit 1,8-Cineol in Form von dünndarmlöslichen Kapseln die in der Literatur berichtete Abnahme der Exazerbationshäufigkeit durch die bekannten kombinierten Therapieverfahren von Budesonid und Formoterol (-24 %, 13) und von Fluticason und Salmeterol (-25 %, 14) noch um weitere 25 % reduziert.

### Zusammenfassung und Ausblick:

Die vorstehend geschilderten Befunde bestätigen eine signifikante Reduktion der inhalativen ICS-Dosis von bis zu 60 % unter Langzeittherapie mit peroralem 1,8-Cineol (Soledum^{®}-Kapseln). Die wesentliche klinische Bedeutung ist die Intensivierung der antiinflammatorischen Wirkung kleinster, in der Peripherie der Lunge abnehmender Konzentrationen gebräuchlicher ICS (z. B. Dosieraerosole und Pulverpräparate).

Der Einsatz von 1,8-Cineol und ICS ist insbesondere zur Therapie der peripher ablaufenden Atemwegsentzündung bei COPD als neues Therapiekonzept und zur Beeinflussung der steroidrefraktären Progression der Lungenerkrankung von Vorteil, um der Entwicklung der irreversiblen respiratorischen Insuffizienz vorzubeugen. Von besonderem klinischem Wert ist beispielsweise der Therapieeinsatz von 1,8-Cineol zur Prophylaxe und Therapie von Frühformen der COPD (d. h. GOLD 0 und GOLD I), für die bisher keine antiinflammatorische Therapie von allen Lungengesellschaften weltweit empfohlen wird. Außerdem wird durch eine kombinierte Therapie von 1,8-Cineol und ICS der steroidrefraktären Atemwegsentzündung durch Zigarettenrauch und auch andere schädliche, proinflammatorische und oxidativ wirksame Umweltstoffe, wie insbesondere Ozon (O₃), und der Entwicklung einer COPD prophylaktisch und therapeutisch entgegengewirkt.

### Beispiel 4: Weitere Versuche und Versuchsdaten

Weiterführende in vitro-Untersuchungen zur antiinflammatorischen Wirkung von 1,8-Cineol, insbesondere bei akuten Erkältungskrankheiten und/oder chronisch-obstruktiven Atemwegserkrankungen

In-vitro-Untersuchungen zur Bestimmung der Effekte einer Zusatztherapie von 1,8-Cineol und inhalierten Steroiden (ICS) auf die Produktion von Superoxiden(O₂⁻) in normalen humanen Monozyten

### Einleitung

Nach dem aktuellen Stand der Forschungsarbeiten konnte die Anmelderin neben entzündungshemmenden auch antioxidative Wirkungen für den Wirkstoff 1,8-Cineol nachweisen. Die antioxidative Wirkung basiert primär auf einer Hemmung von Superoxiden und einer zusätzlichen Wirkung durch Inhibition der Superoxiddismutase-Aktivität (SOD-Aktivität), einhergehend mit einer resultierenden Hemmung von Hydroperoxiden (H₂O₂), die proentzündlich auf die Transkription mit Bildung von Zytokinen und anderen Entzündunsmediatoren wirken.

Systemische 1,8-Cineol, z. B. in Form von Soledum^{®}-Kapseln, kann - wie zuvor ausgeführt- z. B. als Zusatztherapie zunächst bei der schweren COPD (z.B. GOLD III/IV) und/oder als Monotherapie bei leichten Formen (z. B. GOLD I/II), d. h. auch bei chronischer und akuter Bronchitis, eingesetzt werden. Es erscheint insbesondere erwähnenswert, daß die COPD-Leitlinie (GOLD) bei der chronischen Bronchitis außerhalb der Noxenkarenz keine antünflammatorische oder antioxidative Therapie vorschlägt. Dies ist eine Lücke in den aktuellen Leitlinien, die im Rahmen der vorliegenden Anmeldung geschlossen werden konnte.

In den weiterführenden Untersuchungen der Anmelderin zu 1,8-Cineol konnte grundsätzlich die Hypothese verifiziert werden, daß eine Zusatztherapie mit systemischem 1,8-Cineol als Korrelat für bisherige und laufende klinische Untersuchungen mit Soledum^{®}-Kapseln eine potenzierende, überlegene antioxidative und/oder antiinflammatorische Wirkung durch Verstärkung der Mediatorhemmung in normalen humanen Monozyten vermitteln kann.

Da eine antioxidative Wirkung für die Standardtherapie der schweren COPD bestehend aus z. B. LABA plus ICS bisher nicht bekannt ist, wurden im Rahmen der vorliegenden Erfindung erstmals diese neu erkannten Wirkungen von 1,8-Cineol auf die Hemmung von Superoxiden im Vergleich zu ICS und LA-BA (1,8-Cineol vs. ICS und 1,8-Cineol plus ICS) untersucht.

### Hypothese und Fragestellung

Im Vordergrund sollten die antioxidativen Effekte einer Zusatztherapie mit 1,8-Cineol bei entzündlichen Atemwegserkrankungen (z. B. COPD oder Asthma) untersucht werden. Da die Wirkung von 1,8-Cineol als Co-Medikation, insbesondere in Kombination mit inhalativen Steroiden und weiteren Leitlinieneinpfehlungen, noch nicht bekannt ist, besteht ein Erfordernis, neue Grundlagen zur Begründung des neuen Therapieansatzes von 1,8-Cineol bei Atemwegserkrankungen zu entwickeln. Auf der Basis dieser Erkenntnisse sollen vorliegende klinische Studienergebnisse begründet und die Strategie der Zusatztherapie durch ein neues Verständnis etabliert werden. Dies ist zugleich auch die Grundlage zur Prüfung neuer klinischer Fragestellungen zum Einsatz von 1,8-Cineol.

Es soll der zusätzliche Effekt von 1,8-Cineol (4 x 10⁻⁶ mol/l und 6 x 10⁻⁶ mol/l) durch Co-Inkubation mit therapeutisch relevanten Konzentrationen (10⁻¹¹ mol/l, 10⁻¹⁰ mol/l, 10⁻⁹ mol/l) und höheren Konzentrationen (10⁻⁸ mol/l, 10⁻⁷ mol/l, 10⁻⁶ mol/l) von Beclometason ("Becl.") geprüft werden. Außerdem sollen die Effekte von Beclometason allein in den genannten Konzentrationen im Vergleich zu 1,8-Cineol bestimmt werden. Es ist vorgesehen, diese Effekte auf eine Kombination von 1,8-Cineol und Beclometason ("Becl.") als Standard-ICS auf die FKS (fetales Kälberserum)-stimulierte Produktion von O₂⁻ an normalen humanen Monozyten zu prüfen. Ziel der Untersuchungen ist die Prüfung zusätzlicher Effekte einer suboptimalen (4 x 10⁻⁶ mol/l) und optimalen (4 x 10⁻⁶ mol/l) Konzentration von 1,8-Cineol auf unterschiedliche Konzentrationen von Beclometason.

### Methodik

### In-vitro-Verfahren zur Bestimmung der Zusatzeffekte einer Co-Inkubation von 1,8-Cineol und Beclometason

Wie bereits beschrieben, wurden Monozyten aus 50 ml venösem Blut isoliert, das von gesunden, nichtrauchenden Probanden (n = 4) für die Experimente wiederholt freiwillig gespendet wurde. Die Wirkstoffe Beclometason und 1,8-Cineol wurden mit Ethanol bis zu einer maximalen Konzentration von 0,01 % verdünnt. Die Substanzen wurden einzeln (Beclometason 10⁻¹² bis 10⁻⁶ mol/l) und in Kombination (10⁻¹¹ bis 10⁻⁹ mol/l Beclometason mit 6 x 10⁻⁶ mol/l = 0.9 µg/ml 1,8-Cineol) inkubiert sowie in einer Zusatzreihe mit einer nicht antioxidativ wirksamen Konzentration von 1,8-Cineol (4 x 10⁻⁶ mol/l). Hierzu wurden die Wirkstoffe zusammen mit frisch isolierten Monozyten (10⁵/ml) und dem FKS-Stimulus (10 %, Fa. Sigma) für 20 Stunden in einem Zellkulturmittel (RPMI-1640, Fa. Sigma) inkubiert. Dann wurden die Kulturüberstände nach Behandlung der Zellmembranen mit Triton-X 100 gewonnen und sofort auf die Produktion von O₂⁻-Radikalen untersucht.

### Analyseverfahren zur Bestimmung von Superoxiden (O₂⁻) in Kulturüberständen humaner Monozyten

Die Bestimmung der zytosolischen Superoxid-Produktion basiert auf der Reduktion des mit Superoxidionen spezifisch reagierenden Farbstoffs p-Iodonitrotetrazoliumviolett (INTV) zu Iodonitrotetrazoliumformazan (Formazan). INTV wird von den Zellen in das Zytosol aufgenommen. Der Stickstoffring des INTV wird intrazellulär von freien Superoxid-Radikalen reduziert, wobei ein wasserlösliches Zwischenprodukt und das wasserunlösliche Formazan entstehen, die ihr Absorptionsmaximum bei 505 bzw. 490 nm haben. INTV dagegen absorbiert kein Licht dieser Wellenlängen. Am Ende eines Versuchs wird das Zell-Lysat (nach Behandlung mit Salzsäure) bei 492 nm im Photometer vermessen. Hierbei ist die Absorption des Lichts proportional zur intra-zellulär akkumulierten INT-Formazanmenge, welche dann anhand einer Formazan-Eichgraden bestimmt werden kann. Die INT-Formazan-Akkumulation wiederum wird als Maß für die intrazelluläre Superoxid-Produktion bestimmt.

### Kreuzreaktionen mit 1,8-Cineol

Um sicherzustellen, daß tatsächliche Hemmeffekte von 1,8-Cineol auf die INT-Formazan-Akkumulation vorgelegen haben, wurden mögliche Kreuzreaktionen von 1,8-Cineol mit den Bestandteilen des Mess-Systems ausgeschlossen. Dazu wurden Beclometason 10⁻⁶ mol/l, 1,8-Cineol 10⁻⁵mol/l, INTV 0,5 mg/ml sowie Beclometason 10⁻⁶mol/l + 1,8-Cineol 10⁻⁵mol/l + INTV 0,5 mg/ml ohne Zellen inkubiert und die INT-Formazan-Produktion gemessen. Weder 1,8-Cineol oder Beclometason allein oder in Kombination mit INTV hatten einen nachweisbaren Einfluß auf die Bestimmung der INT-Formazan-Produktion. Somit konnten Kreuzreaktionen von 1,8-Cineol und Beclometason mit dem Meßverfahren ausgeschlossen werden.

### Statistische Analysen

Untersuchungen zur dosisabhängigen Produktion von O₂⁻-Radikalen in den unterschiedlichen Fragestellungen erfolgten in Schüsselexperimenten für Beclometason (Expt. 3, n = 10 bis 11), 1,8-Cineol (Expt. 5, n = 12), Beclometason + 1,8-Cineol 4 x 10⁻⁶ mol/l (Expt. 4, n = 14 bis 15) und Beclometason + 1,8-Cineol 6 x 10⁻⁶ mol/l (Expt. 3 , n = 11 bis 12). Effekte von 1,8-Cineol und Beclometason sind als "% der FKS-Kontrolle" ausgedrückt und wurden durch den nichtparametrischen Mann & Whitney-Test statistisch untersucht. P-Werte < 0,05 werden als statistisch signifikant angesehen.

### Ergebnisse

### Vergleich der Effekte von Beclometason und 1,8-Cineol auf die fetale Kälberserum (FKS)-stimulierte Produktion von Superoxiden (O₂⁻) in normalen humanen Monozyten

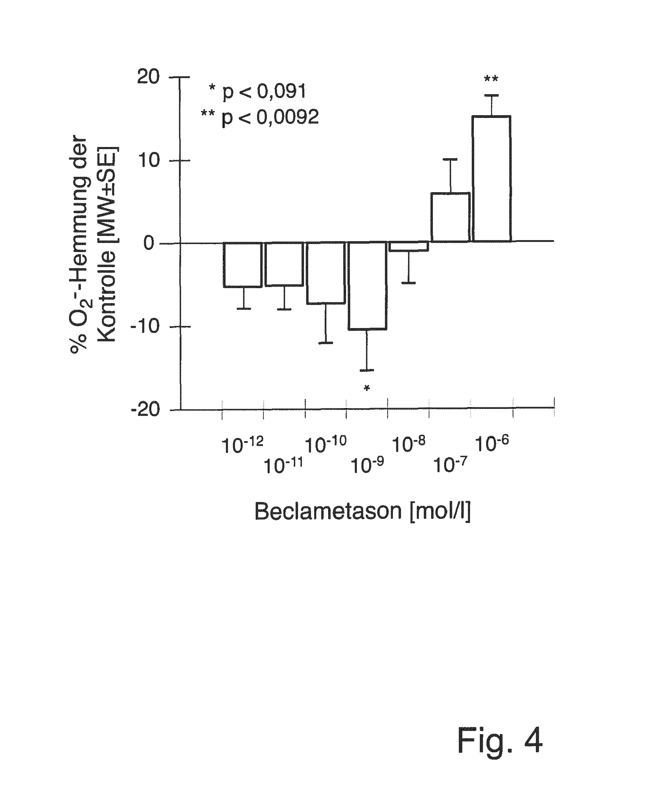

Monozyten (10⁵/ml) wurden mit unterschiedlichen Konzentrationen von Becl.ometason (10⁻¹² bis 10⁻⁶ mol/l, n = 10-11) für 20 Stunden zusammen mit dem FKS-Stimulus (10 %) inkubiert. Nur für Beclometason 10⁻⁹ mol/l wurde eine grenzwertig signifikante Hemmung (-10,5 ± 5 %, p = 0,0910) der O₂⁻-Produktion nachgewiesen (siehe Tabelle 3). Hohe Konzentrationen (≥ 10⁻⁸ mol/l) von Beclometason stimulierten die monozytäre O₂⁻-Produktion mit einer signifikanten Zunahme (15,2 ± 2,5 %, p = 0,0092) bei 10⁻⁶ mol/l (siehe Fig. 4). Somit konnte für Beclometason keine Hemmung der O₂⁻-Produktion nachgewiesen werden.

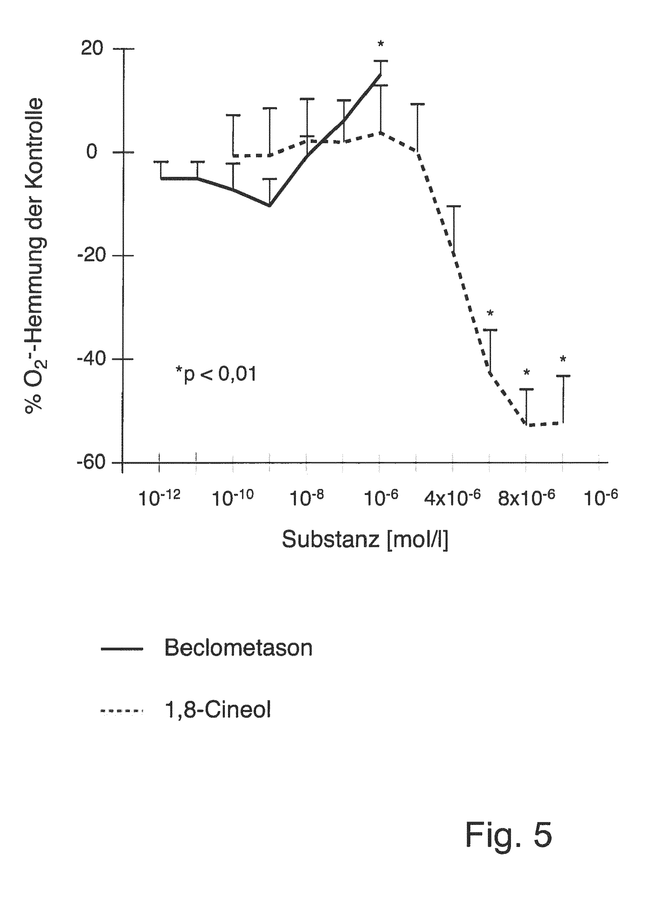

Im Unterschied zur Wirkung von Beclometason konnte bereits eine starke Hemmung der O₂⁻-Produktion (-42,6 ± 8 %, p = 0,0007) für 1,8-Cineol bei einer therapeutisch relevanten Konzentration (0,9 mg/ml =6x10-⁶ mol/l) nachgewiesen werden. Im Unterschied zu Beclometason, das bei therapeutischen Konzentrationen die O₂⁻-Produktion nicht hemmt und bei höheren Konzentrationen stimuliert, hemmte 1,8-Cineol im therapeutischen Bereich die O₂⁻-Produktion. Das Hemmprofil dieser unterschiedlichen Substanzwirkungen ist vergleichend in Fig. 5 dargestellt.

### Effekt einer suboptimalen Konzentration (0,6 µg/ml) von 1,8-Cineol + Beclometason auf die FKS-stimulierte Produktion von Superoxiden (O₂⁻) in normalen Monozyten

Aufgrund der gefundenen unterschiedlichen Wirkungsprofile von Beclometason und 1,8-Cineol sollten additive oder synergistische Wirkungen beider Substanzen gesucht werden. Co-Inkubationen einer selbst noch nicht antioxidativ wirkenden Konzentration von 1,8-Cineol mit Beclometason (n = 14-15) wurden unter den beschrieben experimentellen Vorraussetzungen zur Frage einer synergistischen Wirkung beider Substanzen veranlaßt. Die gewählte Konzentration von 1,8-Cineol 4x10⁻⁶ mol/l zeigte keine Wirkung alleine (-9 ± 6 %, p = 0,2717) wie auch nicht die untersuchten Konzentrationen von Beclometason 10⁻¹¹ bis 10⁻⁹ mol/l (siehe Tabelle 5). Im Unterschied zu den nicht signifikant hemmenden Eigenschaften der Einzelsubstanzen wurde eine ebenfalls nicht signifikante grenzwertige Zunahme der O₂⁻-Produktion für 1,8-Cineol + Beclometason 10⁻¹⁰ mol/l (2± 7 %, p= 0,7557) und für 1,8-Cineol + Beclometason 10⁻⁹ mol/l (4± 8 %, p = 0,8519) nachgewiesen.

**Tabelle 3: Effekt von Beclometason (Becl.) auf die FKS-stimulierte Superoxid-Produktion normaler humaner Monozyten in vitro**

| **mol/l** | **n** | **O₂⁻ (nmol/ml)** | **Prozent der Kontrolle** | **P-Wert** |
|---|---|---|---|---|
| **Spontan** | 11 | 8875 ± 429 | - | - |
| **FKS 10 %** | 11 | 15858 ± 550 | - | <0.0001 |
| **10⁻¹²** | 11 | 15050 ± 455 | -5.1 ± 3 | 0.1783 |
| **10⁻¹¹** | 11 | 15042 + 568 | -5.1 ± 3 | 0.2643 |
| **10⁻¹⁰** | 10 | 14702 ± 682 | -7.3 ± 5 | 0.1392 |
| **10⁻⁹** | 10 | 14192 ± 733 | -10.5 ± 5 | 0.0910 |
| **10⁻⁸** | 10 | 16035 ± 639 | -1.1 ± 4 | 0.7513 |
| **10⁻⁷** | 10 | 16782 ± 678 | +5.8 ± 4 | 0.2751 |
| **10⁻⁶** | 10 | 18240 ± 463 | +15 ± 2.5 | 0.0092 |

**Tabelle 4: Effekt von 1,8-Cineol auf die FKS-stimulierte Superoxid (O₂⁻)-Produktion normaler humaner Monozyten in vitro**

| **1,8-Cineol** | **INT-Formazan** | **Vergleich zur Kontrolle** | **p-Wert** |
|---|---|---|---|
| **Mg/ml (mol/l)** | **(nmol/10⁵)** | **(%)** | |
| **spontan** | **13756 ± 1675** | - | - |
| **FKS 10 %** | **21507 ± 1675** | **56.3 ± 8** | **0.0022** |
| **0.000015 (10⁻¹⁰)** | **21360 ± 1766** | **-0.7 ± 8** | **0.9081** |
| **0.00015 (10⁻⁹)** | **21329 ± 1920** | **-0.8 ± 9** | **0.7728** |
| **0.0015 (10⁻⁸)** | **21946 ± 1858** | **2 ± 8** | **0.7508** |
| **0.015 (10⁻⁷)** | **21899 ± 1879** | **1.8 ± 8** | **0.087** |
| **0.15 (10⁻⁶)** | **22286 ± 2036** | **3.6 ± 9** | **0.4884** |
| **0.3 (2 x 10⁻⁶)** | **21499 ± 1959** | **0.04 ± 9** | **0.9081** |
| **0.6 (4 x 10⁻⁶)** | **17240 ± 1637** | **-19.8 ± 9** | **0.0833** |
| **0.9 (6 x 10⁻⁶)** | **12341 ± 1061** | **-42.6 ± 8** | **0.0007** |
| **1.2 (8 x 10⁻⁶)** | **10086 ± 701** | **-53.1 ± 7** | **< 0.0001** |
| **1.5 (10⁻⁵)** | **10202 ± 915** | **-52.6 ± 9** | **< 0.0001** |

### Effekt eine optimalen Konzentration (0,9 µg/ ml) von 1,8-Cineol + Beclometason auf die FKS-stimulierte Produktion von Superoxiden (O₂⁻) in normalen Monozyten

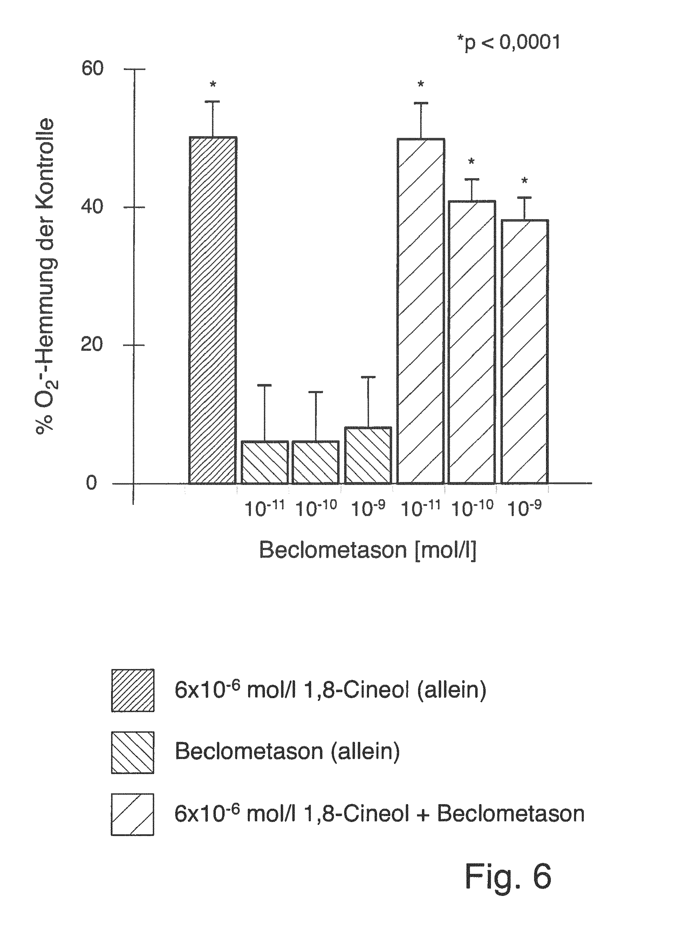

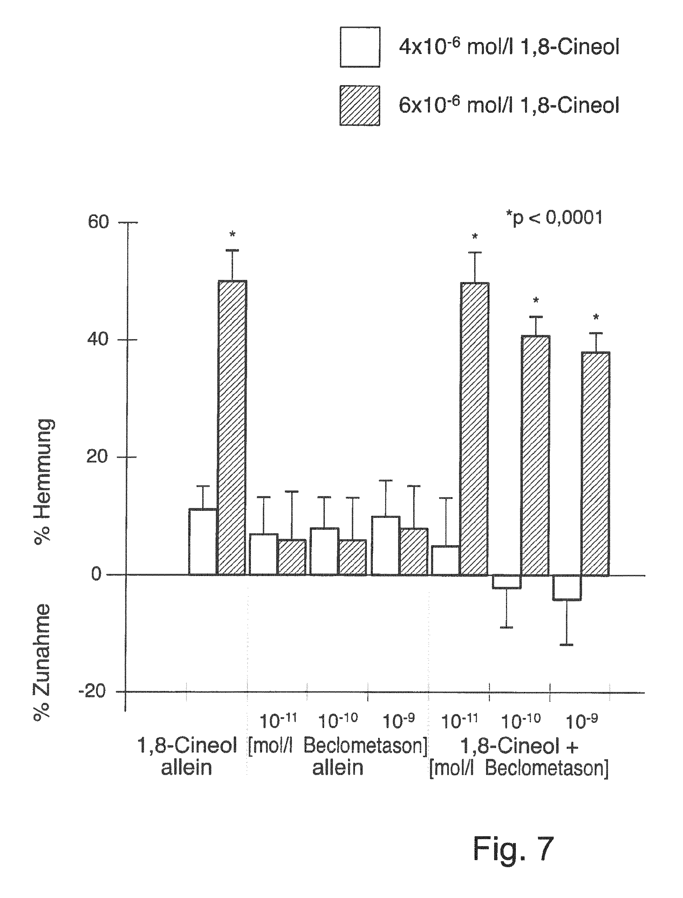

Eine therapeutisch relevante Grenzwertkonzentration von 1,8 Cineol 6 x 10⁻⁶ mol/l (0,9 mg/ ml) wurde als niedrigste antioxidativ wirkende Konzentration für 1,8-Cineol ermittelt und hemmte die O₂⁻ -Produktion signifikant (-50.2± 5 %, < 0,0001). Auch in dieser Experimentreihe hatte Beclometason 10⁻¹¹ bis 10⁻⁹ mol/l allein keinen nachweisbaren Effekt auf die O₂⁻-Produktion (siehe Tabelle 6). Co-Inkubation von 1,8-Cineol + Beclometason 10⁻¹¹ mol/l hemmte die O₂⁻-Produktion (-49,9± 5 %, p < 0,0001), und dieser Effekt ist vergleichbar (p = 0,9215) zur alleinigen Wirkung von 1,8-Cineol. Mit zunehmender Konzentration von Beclometason (10⁻⁹ mol/l) nimmt in Gegenwart von 1,8-Cineol die antioxidative Wirkung tendenziell ab und erreicht -38.1± 3 % (p < 0,0001) (siehe Fig. 6). Statistische Analysen zeigen, daß die Hemmung der O₂⁻-Produktion durch 1,8-Cineol + Beclometason 10⁻¹⁰ mol/l (-40,9± 3 %) signifikant schwächer (p < 0,0001) ist im Vergleich zu 1,8-Cineol allein. Das Gleiche gilt auch für 1,8-Cineol + Beclometason 10⁻⁹ mol/l. Auch ist im Vergleich zu 1,8-Cineol + Beclometason 10⁻¹¹ mol/l die leichtgradig abnehmende Hemmung der O₂⁻-Produktion in Gegenwart höherer Beclometason-Dosen für 1,8-Cineol + Beclometason 10⁻¹⁰mol/L (p = 0,0165) und 1,8-Cineol + Beclometason 10⁻⁹ mol/l (p = 0,0053) signifikant. Diese Daten zeigen, daß die Wirkung von 1,8-Cineol durch die Eigenschaften von Beclometason (10⁻¹⁰ und 10⁻⁹ mol/l) abgeschwächt wird und daß in der Kombination mit Beclometason die führende Rolle der antioxidativen Wirkung von 1,8-Cineol vermittelt wird, die ohne 1,8-Cineol in dieser Kombination nicht nachzuweisen ist (siehe Fig. 7).

**Tabelle 5: Effekt von 1,8-Cineol (0,6 µg/ml) und Beclometason ("Becl.") auf die FKS-stimulierte Superoxid-Produktion normaler humaner Monozyten in vitro**

| **mol/l** | **n** | **O₂⁻ (nmol/ml)** | **Prozent der Kontrolle** | **P-Wert** |
|---|---|---|---|---|
| **spontan** | 15 | 9294 ± 511 | - | - |
| **FKS 10 %** | 15 | 17843 ± 940 | - | < 0.0001 |
| **1,8-Cineol 4x10⁻⁶** | 15 | 16323 ± 1069 | -9 ± 6 | 0.2717 |
| **Becl. 10⁻¹¹** | 14 | 16562 ± 1081 | -7 ± 6 | 0.4581 |
| **Becl. 10⁻¹⁰** | 15 | 16445 ± 903 | -8 ± 5 | 0.2998 |
| **Becl. 10⁻⁹** | 15 | 16052 ± 969 | -10 ± 6 | 0.1466 |
| **1,8-Cineol + Becl. 10⁻¹¹** | 15 | 16867 ± 1399 | -5 ± 8 | 0.4186 |
| **1,8-Cineol + Becl. 10⁻¹⁰** | 15 | 18190 ± 1345 | +2 ± 7 | 0.7557 |
| **1,8-Cineol + Becl. 10⁻⁹** | 15 | 18659 ± 1555 | +4 ± 8 | 0.8519 |

### Zusammenfassende Beurteilung

Die vorliegenden Untersuchungsergebnisse zeigen, daß ein häufig eingesetztes inhalatives Steroid, wie Beclometason, die stimulierte Produktion von O₂⁻-Radikalen in Monozyten nicht hemmt und bei nicht mehr therapeutisch relevanter hoher Konzentration (10⁻⁶mol/l) sogar signifikant steigert. Demgegenüber wird die Produktion von O₂⁻-Radikalen durch therapeutisch relevante Konzentrationen von 1,8-Cineol um ca. 50 % gehemmt. Die aktuelle Untersuchung zeigt daher jetzt erstmalig, daß die starke Hemmung der O₂⁻-Radikalproduktion durch therapeutische Konzentrationen von 1,8-Cineol in der Kombination mit Beclometason nachweisbar ist und einen Vorteil bedeutet, der isoliert durch das inhalative Steroid nicht vermittelt werden kann. Hierbei handelt es sich nicht um einen reinen additiven Effekt, sondern um die Vermittlung der Wirkung von 1,8-Cineol auf die Kombination von 1,8-Cineol und Beclomeatson, ohne daß Beclometason einen eigenständigen antioxidativen Effekt hierbei nachweisen ließe.

Der fehlende Hinweis auf eine Hemmung der O₂⁻-Radikalbildung durch Beclometason ist vermutlich richtungsweisend für die gefundene Stimulierung von O₂⁻-Radikalen durch höhere Konzentrationen von Beclometason, die jedoch nicht therapeutisch relevant sind. Die Ergebnisse zeigen, daß es zwischen Beclometason und 1,8-Cineol eine Interaktion gibt, die bei hohen Dosen von Beclometason die starke Wirkung von 1,8-Cineol negativ-synergistisch abschwächt. Dies ist dadurch zu erkennen, daß 1,8-Cineol allein signifikant stärker antioxidativ wirkt als in Kombination mit Beclometason und selbst die Wirkung von 1,8-Cineol + Beclometason bei steigenden Konzentrationen von Beclometason im Vergleich zu einer Kombination mit kleinerer Beclometason-Konzentration signifikant abnimmt.

Weitere Untersuchungen zeigen im übrigen, daß auch Fluticason die Produktion von O₂⁻-Radikalen induziert, und dies durch eine Hemmung der Superoxiddismutase, die O₂⁻ zu H₂O₂ weitermetabolisiert. Auch nehmen diese Effekte mit Zunahme der Steroidrezeptorbindungsfähigkeit weiter zu, so daß die gegenwärtigen Untersuchungen nahelegen, daß inhalierte Steroide zumindest nicht über die Hemmung von O₂⁻-Radikalen eine antioxidative Wirkung entfalten können und sehr wahrscheinlich gar nicht antioxidativ wirksam sind. Insofern zeigen die Untersuchungen, daß 1,8-Cineol zumindest bezüglich seiner überraschenderweise überlegenen antioxidativen Wirkung gegenüber den heute eingesetzten Steroiden ein bisher unterschätztes vorteilhaftes Wirkprofil in den Atemwegen vermittelt.

Dies allein begründet die Zusatztherapie mit 1,8-Cineol bei entzündlichen Atemwegserkrankungen und läßt in besonderem Maße die Langzeittherapie empfehlen. Da die COPD unter ICS durch Abnahme von Exazerbationen zwar im Verlauf retardiert wird, aber weiterhin unter kombinierter Therapie progredient ist, könnte hier die fehlende antioxidative Wirkung in der gegenwärtig verfügbaren Medikation eine wesentliche Rolle spielen. Der Progreß der COPD wird gerade wesentlich durch Zigarettenrauchen gesteigert, das zu einer hohen Deposition inhalierter O₂⁻-Radikale in den Atemwegen führt, die offenbar nicht durch ICS oder eine kombinierte Therapie mit LABA plus ICS inaktiviert werden können oder die nicht ausreichend protektiv auf Atemwegsepithelzellen und Makrophagen wirksam ist, so daß vorstellbar ist, daß der pathogene Stimulus persistiert und am Progreß der Atemwegserkrankung mitbeteiligt ist.

Ein neuer Aspekt der vorliegenden Erfindung ist auch die bisher unterschätzte Systementzündung bei COPD, die durch das Zigarettenrauchen, aber auch durch die Schwere der Erkrankung induziert wird. Insofern ist vermutlich die inhalative Lokaltherapie nicht ausreichend, um den Verlauf der COPD tatsächlich zu kontrollieren. Ebenso könnten mit den vorliegenden Daten auch die fehlenden Effekte einer Systemtherapie bei COPD mit Prednisolon erklärlich werden, das prooxidativ die Entzündung antreibt in Gegenwart einer nikotininduzierten Steroidresistenz. Zukünftig läßt sich daher ein neuer Stellenwert für die Substanz 1,8-Cineol etablieren. Zudem kann die COPD infolge einer nachgewiesenen lokalen und systemischen Entzündungsreaktion allein durch eine inhalative, kombinierte Therapie nicht ausreichend behandelt werden, so daß sich ein weiteres Standbein für neue Kernaussagen zum Wirkstoff 1,8-Cineol zunehmend entwickelt durch externe Unterstützung weltweiter Forschungsleistungen, die entsprechend umgesetzt werden müssen.

Zusammenfassend eröffnen die vorliegenden Daten eine ganz aktuelle und bisher unterschätzte Perspektive, den neuen Indikationsbereich von 1,8-Cineol auf lokaler Ebene trotz systemischer Applikation zu verstehen. Zugleich wird hierdurch auch eine neue Möglichkeit eröffnet, da die Verträglichkeit infolge von Interaktionen bei verschiedenen Co-Medikationen betrachten müssen. Die eingeschlagene Vorgehensweise wird helfen, die Substanz 1,8-Cineol bezüglich seiner Wirkung und des hieraus abzuleitenden klinischen Einsatzes bei Atemwegserkrankungen richtig zu verstehen.

**Tabelle 6: Additiver Effekt von 1,8-Cineol (0,9 µg/ml) und Beclometason ("Becl.") auf die FKS-stimulierte Superoxid-Produktion normaler humaner Monozyten in vitro**

| **mol/l** | **n** | **O₂⁻ (nmol/ml)** | **Prozent der Kontrolle** | **P-Wert** |
|---|---|---|---|---|
| **spontan** | 11 | 11050 ± 1102 | - | - |
| **FKS 10 %** | 11 | 18778 ± 1178 | - | 0.0009 |
| **1,8-Cineol 6x10⁻⁶** | 11 | 9347 ± 494 | -50.2 ± 5 | < 0.0001 |
| **Becl. 10⁻¹¹** | 11 | 17639 ± 1372 | -6 ± 8 | 0.3088 |
| **Becl. 10⁻¹⁰** | 11 | 17577 ± 1191 | -6 ± 7 | 0.5545 |
| **Becl. 10⁻⁹** | 12 | 17338±1305 | -8± 7 | 0.5767 |
| **1,8-Cineol + Becl. 10⁻¹¹** | 11 | 9395 ±493 | -49.9± 5 | < 0.0001 |
| **1,8-Cineol + Becl. 10⁻¹⁰** | 11 | 11099± 386 | -40.9± 3 | < 0.0001 |
| **1,8-Cineol + Becl. 10⁻⁹** | 11 | 11626 ± 369 | -38.1 ± 3 | < 0.0001 |

Die vorliegende Erfindung wird zudem durch die folgenden Aspekte bzw. Erfindungsgegenstände im Allgemeinen näher beschrieben:

### Aspekt 1:

Verwendung mindestens eines systemisch applizierbaren, insbesondere peroralen Monoterpens und mindestens eines topisch applizierbaren, insbesonderen inhalativen Atemwegstherapeutikums zur prophylaktischen und/oder therapeutischen Behandlung, insbesondere Kombinationstherapie und/oder Co-Medikation, von Atemwegserkrankungen, insbesondere bronchopulmonalen Erkrankungen.

### Aspekt 2:

Verwendung nach Aspekt 1, dadurch gekennzeichnet, daß das Monoterpen ausgewählt ist aus mono- und bicyclischen Monoterpenen, insbesondere aus der Gruppe von monocyclischen Monoterpen-Alkoholen, vorzugsweise Menthol, insbesondere L-Menthol, und bicyclischen Epoxy-Monoterpenen, vorzugsweise Limonenoxiden, bevorzugt 1,8-Cineol, sowie deren Mischungen, besonders bevorzugt Menthol und 1,8-Cineol, ganz besonders bevorzugt 1,8-Cineol, und/oder daß das Monoterpen 1,8-Cineol ist.

### Aspekt 3:

Verwendung nach Aspekt 1 oder 2, dadurch gekennzeichnet, daß das Monoterpen in einer peroralen Darreichungsform, vorzugsweise in Form von Kapseln, vorliegt und/oder daß das Monoterpen in Form einer peroralen, magensaftresistenten, aber dünndarmlöslichen Darreichungsform, insbesondere Kapsel, vorliegt.

### Aspekt 4:

Verwendung nach einem der vorangehenden Aspekte, dadurch gekennzeichnet, daß das Monoterpen in Tagesdosen von 100 bis 2.000 mg/die, insbesondere 200 bis 1.200 mg/die, vorzugsweise 300 bis 1.000 mg/die, verabreicht wird und/oder daß das Monoterpen zur Verabreichung in einer Tagesdosis von 100 bis 2.000 mg/die, insbesondere 200 bis 1.200 mg/die, vorzugsweise 300 bis 1.000 mg/die, hergerichtet ist.

### Aspekt 5:

Verwendung nach einem der vorangehenden Aspekte, dadurch gekennzeichnet, daß das Monoterpen 1,8-Cineol ist, insbesondere wobei 1,8-Cineol in Form von magensaftresistenten, aber dünndarmlöslichen Kapseln eingesetzt wird, vorzugsweise in Tagesdosen von 100 bis 2.000 mg/die, insbesondere 200 bis 1.200 mg/die, besonders bevorzugt 300 bis 1.000 mg/die.

### Aspekt 6:

Verwendung nach einem der vorangehenden Aspekte, dadurch gekennzeichnet, daß das topisch applizierbare Atemwegstherapeutikum ein inhalatives Atemwegstherapeutikum ist.

### Aspekt 7:

Verwendung nach einem der vorangehenden Aspekte, dadurch gekennzeichnet, das topisch applizierbare, insbesondere inhalative Atemwegstherapeutikum ausgewählt ist aus Bronchodilatatoren und Bronchospasmolytika und/oder daß das topisch applizierbare, insbesondere inhalative Atemwegstherapeutikum ausgewählt ist aus der Gruppe von (i) Kortikosteroiden, insbesondere Glukokortikoiden, (ii) Sympathomimetika, insbesondere Betasympathomimetika, vorzugsweise Beta-2-Sympathomimetika; (iii) Phosphodiesterasehemmern; (iv) Parasympatholytika und/oder Vagolytika; (v) Anticholinergika; sowie Mischungen und Kombinationen der vorgenannten Verbindungen, und besonders bevorzugt ausgewählt ist aus der Gruppe von Kortikosteroiden, insbesondere Glukokortikoiden, Beta-2-Sympathomimetika und Anticholinergika sowie deren Mischungen und Kombinationen.

### Aspekt 8:

Verwendung nach einem der vorangehenden Aspekte, dadurch gekennzeichnet, daß das topisch applizierbare, insbesondere inhalative Atemwegstherapeutikum ein Kortikosteroid, insbesondere Glukokortikoid, ist, vorzugsweise ausgewählt aus der Gruppe von Beclometason, Mometason, Budesonid, Flunisolid, Fluticason, Triamcinolon und deren physiologisch verträglichen Derivaten, insbesondere Salzen und Estern, sowie Mischungen und Kombinationen der vorgenannten Verbindungen, insbesondere wobei das Kortikosteroid in Tagesdosen von 50 bis 1.000 µg/die, insbesondere 75 bis 800 µg/die, besonders bevorzugt 100 bis 600 µg/die, verabreicht wird und/oder insbesondere wobei das Kortikosteroid zur Verabreichung in einer Tagesdosis von 50 bis 1.000 µg/die, insbesondere 75 bis 800 µg/die, besonders bevorzugt 100 bis 600 µg/die, hergerichtet ist.

### Aspekt 9:

Verwendung nach einem der vorangehenden Aspekte, dadurch gekennzeichnet, daß das topisch applizierbare, insbesondere inhalative Atemwegstherapeutikum ein Sympathomimetikum, insbesondere Betasympathomimetikum, vorzugsweise Beta-2-Sympathomimetikum, ist, insbesondere ausgewählt aus der Gruppe von kurzwirkenden Betamimetika (SABA), insbesondere Albuterol, Fenoterol, Hexoprenalin, Levalbuterol, Metaproterenol, Orciprenalin, Pirbuterol, Reproterol, Salbutamol und/oder Terbutalin, und langwirkenden Betamimetika (LABA), insbesondere Salmeterol und/oder Formoterol.

### Aspekt 10:

Verwendung nach einem der vorangehenden Aspekte, dadurch gekennzeichnet, daß das topisch applizierbare, insbesondere inhalative Atemwegstherapeutikum ein Anticholinergikum ist, insbesondere aus der Gruppe von Ipratropium, Tiotropium und/oder deren physiologisch verträgliche Derivaten, vorzugsweise Salzen, besonders bevorzugt Ipratropiumbromid und/oder Tiotropiumbromid.

### Aspekt 11:

Verwendung nach einem der vorangehenden Aspekte, dadurch gekennzeichnet, daß zusätzlich mindestens ein weiterer systemischer, insbesondere peroraler Wirkstoff appliziert wird, insbesondere ausgewählt aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; sowie deren Mischungen und Kombinationen.

### Aspekt 12:

Verwendung nach einem der vorangehenden Aspekte, dadurch gekennzeichnet, daß die bronchopulmonale Erkrankung eine entzündliche oder nichtentzündliche, insbesondere entzündliche Erkrankung der oberen oder unteren Atemwege ist.

### Aspekt 13:

Verwendung nach einem der vorangehenden Aspekte, dadurch gekennzeichnet, daß die bronchopulmonale Erkrankung eine entzündliche, insbesondere infektexazerbierte und/oder steroidpflichtige Atemwegserkrankung ist.

### Aspekt 14:

Verwendung nach einem der vorangehenden Aspekte, dadurch gekennzeichnet, daß die bronchopulmonale Erkrankung Asthma bronchiale oder Bronchitis ist.

### Aspekt 15:

Verwendung nach einem der vorangehenden Aspekte, dadurch gekennzeichnet, daß die bronchopulmonale Erkrankung eine chronische obstruktive Lungenerkrankung (COPD) ist, insbesondere eine chronische obstruktive Bronchitis oder ein Lungenemphysem.

### Aspekt 16:

Verwendung nach einem der vorangehenden Aspekte, dadurch gekennzeichnet, daß die bronchopulmonale Erkrankung eine tabkarauchinduzierte, insbesondere nikotininduzierte akute oder chronische Atemwegsentzündung ist.

### Aspekt 17:

Verwendung nach einem der vorangehenden Aspekte, dadurch gekennzeichnet, daß die bronchopulmonale Erkrankung eine Frühform von COPD, insbesondere Stadium nach GOLD 0 oder I, oder eine Frühform des Asthma bronchiale, insbesondere Stadium nach GINA 0 oder I, ist.

### Aspekt 18:

Verwendung nach einem der vorangehenden Aspekte zur Behandlung der Frühform von COPD, insbesondere Stadium nach GOLD 0 oder I, oder der Frühform des Asthma bronchiale, insbesondere Stadium nach GINA 0 oder I, insbesondere zur Exazerbationsprophylaxe vor und nach erfolgter Exazerbation und/oder zur Verhinderung oder Verlangsamung des Krankheitsprogresses vor und nach erfolgter Exazerbation.

### Aspekt 19:

Verwendung mindestens eines systemisch applizierbaren, insbesondere peroralen Monoterpens, vorzugsweise 1,8-Cineol, insbesondere nach einem der vorangehenden Aspekte, zur insbesondere synergistischen Wirkungssteigerung mindestens eines topisch applizierbaren, insbesonderen inhalativen Atemwegstherapeutikums bei der prophylaktischen und/oder therapeutischen Behandlung von Atemwegserkrankungen, insbesondere bronchopulmonalen Erkrankungen.

### Aspekt 20:

Verwendung mindestens eines systemisch applizierbaren, insbesondere peroralen Monoterpens, vorzugsweise 1,8-Cineol, insbesondere nach einem der vorangehenden Aspekte, zur insbesondere synergistischen Steigerung der antiinflammatorischen und/oder antioxidativen Wirkung topischer, insbesondere inhalativer Kortikosteroide, insbesondere Glukokortikoide.

### Aspekt 21:

Verwendung mindestens eines systemisch applizierbaren, insbesondere peroralen Monoterpens, vorzugsweise 1,8-Cineol, insbesondere nach einem der vorangehenden Aspekte, zur Dosisreduktion von topisch applizierbaren, insbesonderen inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

### Aspekt 22:

Verwendung mindestens eines systemisch applizierbaren, insbesondere peroralen Monoterpens, vorzugsweise 1,8-Cineol, insbesondere nach einem der vorangehenden Aspekte, zur Induktion und/oder Verstärkung des steroidpermissiven Effektes von topisch applizierbaren, insbesonderen inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

### Aspekt 23:

Verwendung mindestens eines systemisch applizierbaren, insbesondere peroralen Monoterpens, vorzugsweise 1,8-Cineol, insbesondere nach einem der vorangehenden Aspekte, zur Vermeidung oder Reduktion eines Gewöhnungseffektes von Betasympathomimetika bei der prophylaktischen und/oder therapeutischen Behandlung von Atemwegserkrankungen, insbesondere bronchopulmonalen Erkrankungen, insbesondere unter Dauertherapie bei allen Schweregraden von COPD und Asthma bronchiale.

### Aspekt 24:

Verwendung mindestens eines systemisch applizierbaren, insbesondere peroralen Monoterpens, vorzugsweise 1,8-Cineol, insbesondere nach einem der vorangehenden Aspekte, in Kombination mit mindestens einem topisch applizierbaren, insbesonderen inhalativen Atemwegstherapeutikum, insbesondere in Kombination mit einem inhalativen Kortikosteroid, zur Reduktion des Bedarfs oder zum Ersatz systemischer Kortikosteroide oder anderer antiinflammatorisch und/oder immunsuppressiv wirkender systemischer Substanzen bei der prophylaktischen und/oder therapeutischen Behandlung von Atemwegserkrankungen, insbesondere bronchopulmonalen Erkrankungen.

### Aspekt 25:

Verwendung mindestens eines systemisch applizierbaren, insbesondere peroralen Monoterpens, vorzugsweise 1,8-Cineol, insbesondere nach einem der vorangehenden Aspekte, in Kombination mit mindestens einem topisch applizierbaren, insbesonderen inhalativen Atemwegstherapeutikum, insbesondere in Kombination mit einem inhalativen Kortikosteroid und gegebenenfalls einem inhalativen Beta-2-Sympathomimetikum, zur Optimierung der Basistherapie bei Asthma bronchiale und COPD.

### Aspekt 26:

Verwendung nach einem der vorangehenden Aspekte zur Behandlung der systemischen Organmitbeteiligung bei allen Schwereformen von COPD.

### Aspekt 27:

Verwendung nach einem der vorangehenden Aspekte zur Modulation und Hemmung COPD-abhängiger und/oder COPD-unabhängiger Alterungsprozesse, insbesondere mit dem Ziel der Reduktion von Morbidität, der Erhöhung der Lebensqualität und/oder Lebenserwartung.

### Aspekt 28:

Verwendung nach einem der vorangehenden Aspekte, dadurch gekennzeichnet, daß das Monoterpen, insbesondere 1,8-Cineol, als Induktor der NO-Produktion für die Behandlung der primären und sekundären pulmonalarteriellen Hypertonie (PAH) bei COPD und Asthma bronchiale eingesetzt wird.

### Aspekt 29:

Verwendung nach einem der vorangehenden Aspekte, dadurch gekennzeichnet, daß das Monoterpen, insbesondere 1,8-Cineol, zur Verbesserung der Gewebs- und/oder Mikroperfusion sowie der Bronchodilatation bei NO-Mangelsituationen eingesetzt wird.

### Aspekt 30:

Verwendung nach einem der vorangehenden Aspekte, dadurch gekennzeichnet, daß das Monoterpen, insbesondere 1,8-Cineol, zur Induktion der NO-Produktion bei rezidivierenden Infekten der oberen und unteren Atemwege oder bei infektunabhängigen Exazerbationen, insbesondere durch Zigarettenrauchen und/oder Ozoneinfluß, oder zur Normalisierung noxenabhängiger oder noxenunabhängiger NO-Mangelsituationen eingesetzt wird.

### Aspekt 31:

Verwendung nach einem der vorangehenden Aspekte, dadurch gekennzeichnet, daß das Monoterpen, insbesondere 1,8-Cineol, als Antioxidans und/oder NO-Induktor bei durch Zigarettenrauch induzierten Organschäden, insbesondere der Lunge, des Herzens, des Gehirns, der Nieren und des venösen und arteriellen Gefäßsystems, eingesetzt wird.

### Aspekt 32:

Verwendung nach einem der vorangehenden Aspekte, dadurch gekennzeichnet, daß das Monoterpen, insbesondere 1,8-Cineol, zur kombinierten antiinflammatorischen und/oder antioxidativen Therapie der persistierenden und/oder progredienten Atemwegsentzündung nach Aufgabe des Rauchens, gegebenenfalls unter Co-Medikation mit anderen Atemwegstherapeutika, eingesetzt wird, insbesondere mit dem Ziel der Retardierung der Emphysementwicklung, der respiratorischen Insuffizienz und/oder der Entwicklung peripherer Atemwegsobstruktionen.

### Aspekt 33:

Verwendung nach einem der vorangehenden Aspekte, dadurch gekennzeichnet, daß das Monoterpen, insbesondere 1,8-Cineol, zur Beeinflussung des gesamten Multiorganalterungsprozesses infolge antiinflammatorischer und/oder antioxidativer Wirkeigenschaften durch frühzeitige Langzeittherapie eingesetzt wird.

### Aspekt 34:

Verwendung mindestens eines systemisch applizierbaren, insbesondere peroralen Monoterpens und mindestens eines topisch applizierbaren, insbesondere inhalativen Atemwegstherapeutikums als Kombinationstherapeutikum und/oder zu Zwecken der Co-Medikation zur prophylaktischen und/oder therapeutischen Behandlung von Atemwegserkrankungen, insbesondere bronchopulmonalen Erkrankungen.

### Aspekt 35:

Verwendung mindestens eines systemisch applizierbaren, insbesondere peroralen Monoterpens und mindestens eines topisch applizierbaren, insbesondere inhalativen Atemwegstherapeutikums zur Herstellung eines Kombinationstherapeutikums, insbesondere in Form eines Kit, zur prophylaktischen und/oder therapeutischen Behandlung von Atemwegserkrankungen, insbesondere bronchopulmonalen Erkrankungen.

### Aspekt 36:

Kombinationstherapeutikum, insbesondere in Form eines Kit, vorzugsweise zur prophylaktischen und/oder therapeutischen Behandlung von Atemwegserkrankungen, insbesondere bronchopulmonalen Erkrankungen, umfassend mindestens ein systemisch applizierbares, insbesondere perorales Monoterpen einerseits und mindestens ein topisch applizierbares, insbesondere inhalatives Atemwegstherapeutikum andererseits.

### Aspekt 37:

Verfahren zur Behandlung des menschlichen oder tierischen Körpers, insbesondere Verfahren zur prophylaktischen und/oder therapeutischen Behandlung von Atemwegserkrankungen, insbesondere bronchopulmonalen Erkrankungen, wobei mindestens ein Monoterpen und mindestens ein Atemwegstherapeutikum in jeweils wirksamen, insbesondere pharmazeutisch wirksamen Mengen eingesetzt werden, wobei das Monoterpen systemisch, insbesondere peroral appliziert wird und das Atemwegstherapeutikum topisch, insbesondere inhalativ appliziert wird.

### Aspekt 38:

Verwendung mindestens eines systemisch applizierbaren, insbesondere peroralen Monoterpens, vorzugsweise 1,8-Cineol, zur prophylaktischen und/oder kurativen Behandlung von Atemwegserkrankungen, insbesondere bronchopulmonalen Erkrankungen, bei Rauchern oder Exrauchern bzw. zur Herstellung eines Arzneimittels zur prophylaktischen und/oder kurativen Behandlung von Atemwegserkrankungen, insbesondere bronchopulmonalen Erkrankungen, bei Rauchern oder Exrauchern, insbesondere wobei das Monoterpen zusammen mit mindestens einem topisch applizierbaren, insbesonderen inhalativen Atemwegstherapeutikum, insbesondere als Kombinationstherapie und/oder in Co-Medikation, eingesetzt werden kann.

### Aspekt 39:

Verwendung mindestens eines systemisch applizierbaren, insbesondere peroralen Monoterpens, vorzugsweise 1,8-Cineol, zur prophylaktischen und/oder kurativen Behandlung antioxidativer und/oder antiinflammatorischer Prozesse im Körper insbesondere von Rauchern bzw. zur Herstellung eines Arzneimittels zur prophylaktischen und/oder kurativen Behandlung antioxidativer und/oder antiinflammatorischer Prozesse im Körper insbesondere von Rauchern, insbesondere wobei das Monoterpen zusammen mit mindestens einem topisch applizierbaren, insbesonderen inhalativen Atemwegstherapeutikum, insbesondere als Kombinationstherapie und/oder in Co-Medikation, eingesetzt werden kann.

### Aspekt 40:

Verwendung mindestens eines systemisch applizierbaren, insbesondere peroralen Monoterpens, vorzugsweise 1,8-Cineol, zur Erhöhung der Kortikosteroidsensitivität von Rauchern, insbesondere zur prophylaktischen und/oder therapeutischen Behandlung von Atemwegserkrankungen, insbesondere bronchopulmonalen Erkrankungen, insbesondere wobei das Monoterpen zusammen mit mindestens einem topisch applizierbaren, insbesonderen inhalativen Atemwegstherapeutikum, insbesondere als Kombinationstherapie und/oder in Co-Medikation, eingesetzt werden kann.

### Aspekt 41:

Verwendung nach einem der Aspekte 38 bis 40, dadurch gekennzeichnet, daß das Monoterpen ausgewählt ist aus mono- und bicyclischen Monoterpenen, insbesondere aus der Gruppe von monocyclischen Monoterpen-Alkoholen, vorzugsweise Menthol, insbesondere L-Menthol, und bicyclischen Epoxy-Monoterpenen, vorzugsweise Limonenoxiden, bevorzugt 1,8-Cineol, sowie deren Mischungen, besonders bevorzugt Menthol und 1,8-Cineol, ganz besonders bevorzugt 1,8-Cineol, und/oder daß das Monoterpen 1,8-Cineol ist und/oder daß das Monoterpen in einer peroralen Darreichungsform, vorzugsweise in Form von Kapseln, vorliegt und/oder daß das Monoterpen in Form einer peroralen, magensaftresistenten, aber dünndarmlöslichen Darreichungsform, insbesondere Kapsel, vorliegt und/oder daß das Monoterpen in Tagesdosen von 100 bis 2.000 mg/die, insbesondere 200 bis 1.200 mg/die, vorzugsweise 300 bis 1.000 mg/die, verabreicht wird und/oder daß das Monoterpen zur Verabreichung in einer Tagesdosis von 100 bis 2.000 mg/die, insbesondere 200 bis 1.200 mg/die, vorzugsweise 300 bis 1.000 mg/die, hergerichtet ist.

### Aspekt 42:

Verwendung nach einem der Aspekte 38 bis 41, gekennzeichnet durch eines oder mehrere der Merkmale der Aspekte 1 bis 37.

## Patentansprüche

1. Kombinationstherapeutikum, insbesondere in Form eines Kit, vorzugsweise zur prophylaktischen und/oder therapeutischen Behandlung von Atemwegserkrankungen, insbesondere bronchopulmonalen Erkrankungen, umfassend mindestens ein systemisch applizierbares, insbesondere perorales Monoterpen einerseits und mindestens ein topisch applizierbares, insbesondere inhalatives Atemwegstherapeutikum andererseits.

2. Kombinationstherapeutikum nach Anspruch 1, **dadurch gekennzeichnet, daß** mindestens ein Monoterpen und mindestens ein Atemwegstherapeutikum in jeweils wirksamen, insbesondere pharmazeutisch wirksamen Mengen eingesetzt werden, wobei das Monoterpen systemisch, insbesondere peroral appliziert wird und das Atemwegstherapeutikum topisch, insbesondere inhalativ appliziert wird; und/oder
daß das Monoterpen ausgewählt ist aus mono- und bicyclischen Monoterpenen, insbesondere aus der Gruppe von monocyclischen Monoterpen-Alkoholen, vorzugsweise Menthol, insbesondere L-Menthol, und bicyclischen Epoxy-Monoterpenen, vorzugsweise Limonenoxiden, bevorzugt 1,8-Cineol, sowie deren Mischungen, besonders bevorzugt Menthol und 1,8-Cineol, ganz besonders bevorzugt 1,8-Cineol, und/oder daß das Monoterpen 1,8-Cineol ist; und/oder
daß das Monoterpen in einer peroralen Darreichungsform, vorzugsweise in Form von Kapseln, vorliegt und/oder daß das Monoterpen in Form einer peroralen, magensaftresistenten, aber dünndarmlöslichen Darreichungsform, insbesondere Kapsel, vorliegt; und/oder
daß das Monoterpen in Tagesdosen von 100 bis 2.000 mg/die, insbesondere 200 bis 1.200 mg/die, vorzugsweise 300 bis 1.000 mg/die, verabreicht wird und/oder daß das Monoterpen zur Verabreichung in einer Tagesdosis von 100 bis 2.000 mg/die, insbesondere 200 bis 1.200 mg/die, vorzugsweise 300 bis 1.000 mg/die, hergerichtet ist.

3. Kombinationstherapeutikum nach Anspruch 1 oder 2, **dadurch gekennzeichnet,**
**daß** das topisch applizierbare Atemwegstherapeutikum ein inhalatives Atemwegstherapeutikum ist; und/oder
**daß** das topisch applizierbare, insbesondere inhalative Atemwegstherapeutikum ausgewählt ist aus Bronchodilatatoren und Bronchospasmolytika und/oder daß das topisch applizierbare, insbesondere inhalative Atemwegstherapeutikum ausgewählt ist aus der Gruppe von (i) Kortikosteroiden, insbesondere Glukokortikoiden, (ii) Sympathomimetika, insbesondere Betasympathomimetika, vorzugsweise Beta-2-Sympathomimetika; (iii) Phosphodiesterasehemmern; (iv) Parasympatholytika und/oder Vagolytika; (v) Anticholinergika; sowie Mischungen und Kombinationen der vorgenannten Verbindungen, und besonders bevorzugt ausgewählt ist aus der Gruppe von Kortikosteroiden, insbesondere Glukokortikoiden, Beta-2-Sympathomimetika und Anticholinergika sowie deren Mischungen und Kombinationen; und/oder
**daß** das topisch applizierbare, insbesondere inhalative Atemwegstherapeutikum ein Kortikosteroid, insbesondere Glukokortikoid, ist, vorzugsweise ausgewählt aus der Gruppe von Beclometason, Mometason, Budesonid, Flunisolid, Fluticason, Triamcinolon und deren physiologisch verträglichen Derivaten, insbesondere Salzen und Estern, sowie Mischungen und Kombinationen der vorgenannten Verbindungen, insbesondere wobei das Kortikosteroid in Tagesdosen von 50 bis 1.000 µg/die, insbesondere 75 bis 800 µg/die, besonders bevorzugt 100 bis 600 µg/die, verabreicht wird und/oder insbesondere wobei das Kortikosteroid zur Verabreichung in einer Tagesdosis von 50 bis 1.000 µg/die, insbesondere 75 bis 800 µg/die, besonders bevorzugt 100 bis 600 µg/die, hergerichtet ist; und/oder
**daß** das topisch applizierbare, insbesondere inhalative Atemwegstherapeutikum ein Sympathomimetikum, insbesondere Betasympathomimetikum, vorzugsweise Beta-2-Sympathomimetikum, ist, insbesondere ausgewählt aus der Gruppe von kurzwirkenden Betamimetika (SABA), insbesondere Albuterol, Fenoterol, Hexoprenalin, Levalbuterol, Metaproterenol, Orciprenalin, Pirbuterol, Reproterol, Salbutamol und/oder Terbutalin, und langwirkenden Betamimetika (LABA), insbesondere Salmeterol und/oder Formoterol; und/oder
**daß** das topisch applizierbare, insbesondere inhalative Atemwegstherapeutikum ein Anticholinergikum ist, insbesondere aus der Gruppe von Ipratropium, Tiotropium und/oder deren physiologisch verträgliche Derivaten, vorzugsweise Salzen, besonders bevorzugt Ipratropiumbromid und/oder Tiotropiumbromid.

4. Kombinationstherapeutikum nach einem der vorangehenden Ansprüche, **dadurch gekennzeichnet, daß** zusätzlich mindestens ein weiterer systemischer, insbesondere peroraler Wirkstoff appliziert wird, insbesondere ausgewählt aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; sowie deren Mischungen und Kombinationen.

5. Kombinationstherapeutikum nach einem der vorangehenden Ansprüche, **dadurch gekennzeichnet,**
**daß** die bronchopulmonale Erkrankung eine entzündliche oder nichtentzündliche, insbesondere entzündliche Erkrankung der oberen oder unteren Atemwege ist; und/oder
**daß** die bronchopulmonale Erkrankung eine entzündliche, insbesondere infektexazerbierte und/oder steroidpflichtige Atemwegserkrankung ist; und/oder
**daß** die bronchopulmonale Erkrankung Asthma bronchiale oder Bronchitis ist; und/oder
**daß** die bronchopulmonale Erkrankung eine chronische obstruktive Lungenerkrankung (COPD) ist, insbesondere eine chronische obstruktive Bronchitis oder ein Lungenemphysem; und/oder
**daß** die bronchopulmonale Erkrankung eine tabkarauchinduzierte, insbesondere nikotininduzierte akute oder chronische Atemwegsentzündung ist; und/oder
**daß** die bronchopulmonale Erkrankung eine Frühform von COPD, insbesondere Stadium nach GOLD 0 oder I, oder eine Frühform des Asthma bronchiale, insbesondere Stadium nach GINA 0 oder I, ist.

6. Kombinationstherapeutikum nach einem der vorangehenden Ansprüche
zur insbesondere synergistischen Wirkungssteigerung mindestens eines topisch applizierbaren, insbesonderen inhalativen Atemwegstherapeutikums bei der prophylaktischen und/oder therapeutischen Behandlung von Atemwegserkrankungen, insbesondere bronchopulmonalen Erkrankungen; und/oder
zur insbesondere synergistischen Steigerung der antiinflammatorischen und/oder antioxidativen Wirkung topischer, insbesondere inhalativer Kortikosteroide, insbesondere Glukokortikoide; und/oder
zur Dosisreduktion von topisch applizierbaren, insbesonderen inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen; und/oder
zur Induktion und/oder Verstärkung des steroidpermissiven Effektes von topisch applizierbaren, insbesonderen inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen; und/oder
zur Vermeidung oder Reduktion eines Gewöhnungseffektes von Betasympathomimetika bei der prophylaktischen und/oder therapeutischen Behandlung von Atemwegserkrankungen, insbesondere bronchopulmonalen Erkrankungen, insbesondere unter Dauertherapie bei allen Schweregraden von COPD und Asthma bronchiale.
zur Reduktion des Bedarfs oder zum Ersatz systemischer Kortikosteroide oder anderer antiinflammatorisch und/oder immunsuppressiv wirkender systemischer Substanzen bei der prophylaktischen und/oder therapeutischen Behandlung von Atemwegserkrankungen, insbesondere bronchopulmonalen Erkrankungen.

7. Verwendung mindestens eines systemisch applizierbaren, insbesondere peroralen Monoterpens und mindestens eines topisch applizierbaren, insbesonderen inhalativen Atemwegstherapeutikums zur prophylaktischen und/oder therapeutischen Behandlung, insbesondere Kombinationstherapie und/oder Co-Medikation, von Atemwegserkrankungen, insbesondere bronchopulmonalen Erkrankungen.

8. Verwendung nach Anspruch 7, **dadurch gekennzeichnet,**
**daß** das Monoterpen ausgewählt ist aus mono- und bicyclischen Monoterpenen, insbesondere aus der Gruppe von monocyclischen Monoterpen-Alkoholen, vorzugsweise Menthol, insbesondere L-Menthol, und bicyclischen Epoxy-Monoterpenen, vorzugsweise Limonenoxiden, bevorzugt 1,8-Cineol, sowie deren Mischungen, besonders bevorzugt Menthol und 1,8-Cineol, ganz besonders bevorzugt 1,8-Cineol, und/oder daß das Monoterpen 1,8-Cineol ist; und/oder
**daß** das Monoterpen in einer peroralen Darreichungsform, vorzugsweise in Form von Kapseln, vorliegt und/oder daß das Monoterpen in Form einer peroralen, magensaftresistenten, aber dünndarmlöslichen Darreichungsform, insbesondere Kapsel, vorliegt; und/oder
**daß** das Monoterpen in Tagesdosen von 100 bis 2.000 mg/die, insbesondere 200 bis 1.200 mg/die, vorzugsweise 300 bis 1.000 mg/die, verabreicht wird und/oder daß das Monoterpen zur Verabreichung in einer Tagesdosis von 100 bis 2.000 mg/die, insbesondere 200 bis 1.200 mg/die, vorzugsweise 300 bis 1.000 mg/die, hergerichtet ist; und/oder
**daß** das Monoterpen 1,8-Cineol ist, insbesondere wobei 1,8-Cineol in Form von magensaftresistenten, aber dünndarmlöslichen Kapseln eingesetzt wird, vorzugsweise in Tagesdosen von 100 bis 2.000 mg/die, insbesondere 200 bis 1.200 mg/die, besonders bevorzugt 300 bis 1.000 mg/die.

9. Verwendung nach Anspruch 7 oder 8, **dadurch gekennzeichnet,**
**daß** das topisch applizierbare Atemwegstherapeutikum ein inhalatives Atemwegstherapeutikum ist; und/oder
das topisch applizierbare, insbesondere inhalative Atemwegstherapeutikum ausgewählt ist aus Bronchodilatatoren und Bronchospasmolytika und/oder daß das topisch applizierbare, insbesondere inhalative Atemwegstherapeutikum ausgewählt ist aus der Gruppe von (i) Kortikosteroiden, insbesondere Glukokortikoiden, (ii) Sympathomimetika, insbesondere Betasympathomimetika, vorzugsweise Beta-2-Sympathomimetika; (iii) Phosphodiesterasehemmern; (iv) Parasympatholytika und/oder Vagolytika; (v) Anticholinergika; sowie Mischungen und Kombinationen der vorgenannten Verbindungen, und besonders bevorzugt ausgewählt ist aus der Gruppe von Kortikosteroiden, insbesondere Glukokortikoiden, Beta-2-Sympathomimetika und Anticholinergika sowie deren Mischungen und Kombinationen; und/oder
**daß** das topisch applizierbare, insbesondere inhalative Atemwegstherapeutikum ein Kortikosteroid, insbesondere Glukokortikoid, ist, vorzugsweise ausgewählt aus der Gruppe von Beclometason, Mometason, Budesonid, Flunisolid, Fluticason, Triamcinolon und deren physiologisch verträglichen Derivaten, insbesondere Salzen und Estern, sowie Mischungen und Kombinationen der vorgenannten Verbindungen, insbesondere wobei das Kortikosteroid in Tagesdosen von 50 bis 1.000 µg/die, insbesondere 75 bis 800 µg/die, besonders bevorzugt 100 bis 600 µg/die, verabreicht wird und/oder insbesondere wobei das Kortikosteroid zur Verabreichung in einer Tagesdosis von 50 bis 1.000 µg/die, insbesondere 75 bis 800 µg/die, besonders bevorzugt 100 bis 600 µg/die, hergerichtet ist; und/oder
**daß** das topisch applizierbare, insbesondere inhalative Atemwegstherapeutikum ein Sympathomimetikum, insbesondere Betasympathomimetikum, vorzugsweise Beta-2-Sympathomimetikum, ist, insbesondere ausgewählt aus der Gruppe von kurzwirkenden Betamimetika (SABA), insbesondere Albuterol, Fenoterol, Hexoprenalin, Levalbuterol, Metaproterenol, Orciprenalin, Pirbuterol, Reproterol, Salbutamol und/oder Terbutalin, und langwirkenden Betamimetika (LABA), insbesondere Salmeterol und/oder Formoterol; und/oder
**daß** das topisch applizierbare, insbesondere inhalative Atemwegstherapeutikum ein Anticholinergikum ist, insbesondere aus der Gruppe von Ipratropium, Tiotropium und/oder deren physiologisch verträgliche Derivaten, vorzugsweise Salzen, besonders bevorzugt Ipratropiumbromid und/oder Tiotropiumbromid; und/oder
**daß** zusätzlich mindestens ein weiterer systemischer, insbesondere peroraler Wirkstoff appliziert wird, insbesondere ausgewählt aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; sowie deren Mischungen und Kombinationen.

10. Verwendung nach einem der vorangehenden Ansprüche, **dadurch gekennzeichnet,**
**daß** die bronchopulmonale Erkrankung eine entzündliche oder nichtentzündliche, insbesondere entzündliche Erkrankung der oberen oder unteren Atemwege ist; und/oder
**daß** die bronchopulmonale Erkrankung eine entzündliche, insbesondere infektexazerbierte und/oder steroidpflichtige Atemwegserkrankung ist; und/oder
**daß** die bronchopulmonale Erkrankung Asthma bronchiale oder Bronchitis ist; und/oder
**daß** die bronchopulmonale Erkrankung eine chronische obstruktive Lungenerkrankung (COPD) ist, insbesondere eine chronische obstruktive Bronchitis oder ein Lungenemphysem; und/oder
**daß** die bronchopulmonale Erkrankung eine tabkarauchinduzierte, insbesondere nikotininduzierte akute oder chronische Atemwegsentzündung ist; und/oder
**daß** die bronchopulmonale Erkrankung eine Frühform von COPD, insbesondere Stadium nach GOLD 0 oder I, oder eine Frühform des Asthma bronchiale, insbesondere Stadium nach GINA 0 oder I, ist.

11. Verwendung nach einem der vorangehenden Ansprüche zur Behandlung der Frühform von COPD, insbesondere Stadium nach GOLD 0 oder I, oder der Frühform des Asthma bronchiale, insbesondere Stadium nach GINA 0 oder I, insbesondere zur Exazerbationsprophylaxe vor und nach erfolgter Exazerbation und/oder zur Verhinderung oder Verlangsamung des Krankheitsprogresses vor und nach erfolgter Exazerbation.

12. Verwendung mindestens eines systemisch applizierbaren, insbesondere peroralen Monoterpens, vorzugsweise 1,8-Cineol, insbesondere nach einem der vorangehenden Ansprüche,
zur insbesondere synergistischen Wirkungssteigerung mindestens eines topisch applizierbaren, insbesonderen inhalativen Atemwegstherapeutikums bei der prophylaktischen und/oder therapeutischen Behandlung von Atemwegserkrankungen, insbesondere bronchopulmonalen Erkrankungen; oder
zur insbesondere synergistischen Steigerung der antiinflammatorischen und/oder antioxidativen Wirkung topischer, insbesondere inhalativer Kortikosteroide, insbesondere Glukokortikoide; oder
zur Dosisreduktion von topisch applizierbaren, insbesonderen inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen; oder
zur Induktion und/oder Verstärkung des steroidpermissiven Effektes von topisch applizierbaren, insbesonderen inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen; oder
zur Vermeidung oder Reduktion eines Gewöhnungseffektes von Betasympathomimetika bei der prophylaktischen und/oder therapeutischen Behandlung von Atemwegserkrankungen, insbesondere bronchopulmonalen Erkrankungen, insbesondere unter Dauertherapie bei allen Schweregraden von COPD und Asthma bronchiale.

13. Verwendung mindestens eines systemisch applizierbaren, insbesondere peroralen Monoterpens, vorzugsweise 1,8-Cineol, insbesondere nach einem der vorangehenden Ansprüche, in Kombination mit mindestens einem topisch applizierbaren, insbesonderen inhalativen Atemwegstherapeutikum, insbesondere in Kombination mit einem inhalativen Kortikosteroid, zur Reduktion des Bedarfs oder zum Ersatz systemischer Kortikosteroide oder anderer antiinflammatorisch und/oder immunsuppressiv wirkender systemischer Substanzen bei der prophylaktischen und/oder therapeutischen Behandlung von Atemwegserkrankungen, insbesondere bronchopulmonalen Erkrankungen.

14. Verwendung mindestens eines systemisch applizierbaren, insbesondere peroralen Monoterpens, vorzugsweise 1,8-Cineol, insbesondere nach einem der vorangehenden Ansprüche, in Kombination mit mindestens einem topisch applizierbaren, insbesonderen inhalativen Atemwegstherapeutikum, insbesondere in Kombination mit einem inhalativen Kortikosteroid und gegebenenfalls einem inhalativen Beta-2-Sympathomimetikum, zur Optimierung der Basistherapie bei Asthma bronchiale und COPD.

15. Verwendung mindestens eines systemisch applizierbaren, insbesondere peroralen Monoterpens und mindestens eines topisch applizierbaren, insbesondere inhalativen Atemwegstherapeutikums zur Herstellung eines Kombinationstherapeutikums, insbesondere in Form eines Kit, zur prophylaktischen und/oder therapeutischen Behandlung von Atemwegserkrankungen, insbesondere bronchopulmonalen Erkrankungen.
